(19) Europäisches Patentamt
European Patent Office
Office européen des brevets

(11) Publication number: 0 130 047
A1

# (12) EUROPEAN PATENT APPLICATION

(21) Application number: 84304191.4

(22) Date of filing: 21.06.84

(51) Int. Cl.[4]: C 12 N 15/00
//C12R1/41

(30) Priority: 22.06.83 US 506676

(43) Date of publication of application:
02.01.85 Bulletin 85/1

(84) Designated Contracting States:
AT BE CH DE FR GB IT LI LU NL SE

(71) Applicant: Agrigenetics Research Associates Limited
3375 Mitchell Lane
Boulder Colorado(US)

(72) Inventor: Shine, John
107 Barnett Close
Swinger Hill A.C.T. 2606 Canberra(AU)

(72) Inventor: Rolfe, Barry G.
84 Morgan
Curtin A.C.T. 2605 Canberra(AU)

(72) Inventor: Scott, Kieran F.
29A Hillebrand Street
Page A.C.T. 2614 Canberra(AU)

(74) Representative: De Minvielle-Devaux, Ian Benedict Peter et al,
CARPMAELS & RANSFORD 43, Bloomsbury Square
London WC1A 2RA(GB)

## (54) NIF genes.

(57) There is provided a bacterial strain containing and replicating therein a recombinant DNA plasmid comprising:

(a) a vector

(b) a fragment of DNA controlling expression of a nitrogenase complex structural gene, and

(c) a structural gene under control of said fragment of DNA.

EP 0 130 047 A1

# NIF GENES

## Field of the Invention

Biological nitrogen fixation in the root nodules of leguminous plants is a major component of world food production and therefore practical applications of this field are of major interest.

Prokaryotes can use a wide variety of nitrogen compounds as sole sources of cellular nitrogen. This variety includes ammonia, dinitrogen and nitrate among the inorganic compounds, and proline, arginine and glutamine among complex organic compounds. Each species can utilize a different array of nitrogen compounds. Glutamine, glutamate and aspartate are the key nitrogen compounds in intermediary metabolism. The latter two are the starting compounds of many pathways of amino acid biosynthesis and serve as amino group donors in many reactions. In all other cases the amino group is donated by glutamine. The major enzyme required for the assimilation of ammonia is glutamine synthetase, which catalyses the reaction:

Glutamate + $NH_3$ + ATP ----> glutamine + ADP + Pi.

Utilization of the assimilated ammonia depends on the activity of glutamate synthase catalyzing:

Glutamine + 2-ketoglutarate + NADPH ----> 2 glutamate + $NADP^+$

Since ATP is hydrolysed, these reactions have a favorable equilibrium and allow the use of ammonia in the medium or ammonia derived enzymatically from other nitrogen sources (Meers, J., Tempest, D. and C. Brown (1970) J. Gen. Microbiol. 64:187-194). The formation of ammonia is thus a key step in the biological nitrogen cycle.

Biological nitrogen fixation can be achieved by a variety of microorganisms and occurs through the induction of an enzyme complex, nitrogenase, which converts atmospheric nitrogen to ammonia. This conversion

occurs in a group of physiologically diverse prokaryotes, including facultative anaerobes (e.g., Klebsiella pneumoniae) obligate aerobes (e.g., Azotobacter vinelandii), photosynthetic bacteria (e.g., Rhodospirillum rubrum),and some strains of blue-green algae (e.g., Anabaena cylindrica) (Sprent, J. I. (1979) The biology of nitrogen fixing organisms, London, McGraw-Hill, pp. 8-11). While this enzyme complex is common to all characterized nitrogen fixing organisms, the conditions under which it is expressed vary considerably between species (Burns, R. C., Hardy, R. W. F. (1975): Nitrogen fixation in bacteria and higher plants, Springer-Verlag, Berlin). The first stages of nitrogen fixation, conversion of nitrogen into ammonia, are achieved symbiotically in the root nodules of leguminous plants which contain the nitrogen-fixing bacteria of the genus Rhizobium. Some non-leguminous plants, e.g., alder, also have interactions with symbiotic bacteria which are nitrogen fixers. In addition, free-living bacteria, e.g., Klebsiella pneumoniae and the photosynthetic blue-green bacteria, also fix nitrogen. Biological nitrogen fixation in the root nodules of leguminous plants is a major component of world food production (Burris, R. H. (1980) In Free Living Systems and Chemical Models; Nitrogen fixation, Newton, W. E., Orne-Johnson, W. H., eds. Vol 1 Baltimore, University Park Press, pp. 7-16).

The symbiotic association between plants and bacteria of the genus Rhizobium is the result of a complex interaction between the bacterium and its host, requiring the expression of both bacterial and plant genes in a tightly coordinated manner (Vincent, J. M. (1980) In Symbiotic Associations and Cyanobacteria, Nitrogen Fixation Vol. 2 (W. E. Newton, W. H. Orne-Johnson, eds. Baltimore, University Park Press pp. 103-129; and Verma, D. P. S., Legocki, R. P. and S. Auger (1981) In Current Perspectives in Nitrogen Fixation (A. H. Gibson, W. E. Newton, eds.) Canberra: Australian Academy of Science, pp. 205-208). In free-living Rhizobia, nitrogenase synthesis is repressed and is only induced after the symbiotic relationship has been established. Furthermore, some Rhizobium species only interact with a narrow range of plant species, whereas other species interact with a wide range.

Bacteria bind to the emerging plant root hairs and invade the root tissue through the formation of an infection thread. The plant responds to this infection by the development of a highly differentiated root nodule. These nodules are the site of synthesis of the nitrogenase complex. Following

nitrogen fixation, the fixed nitrogen is exported into the plant tissue and assimilated by the plant derived enzymes (Scott, D. B., Farnden, K. J. F. and Robertson, J. G. (1976) Nature 263:703,705).

Most Rhizobium symbioses are confined to leguminous plants. Furthermore, Rhizobium strains which fix nitrogen in association with the agriculturally-important temperate legumes are usually restricted in their host range to a single legume genus. However, some strains of Rhizobium have been isolated which can fix nitrogen in a diverse group of legume species but can also form an effective symbiosis with non-legumes.

Despite the ability of certain plants to induce nitrogenase activity in a symbiotic relationship with some species of Rhizobium, the genetic analysis of biological nitrogen fixation has previously been confined to free living nitrogen fixing organisms, in particular Klebsiella pneumoniae. There are 17 linked nitrogen fixation (nif) genes arranged in at least 7 transcriptional units in the nif cluster of Klebsiella (Kennedy, C., Cannon, F., Cannon, M., Dixon, R., Hill, S., Jensen, J., Kumar, S., McLean, P., Merrick, M., Robson, R. and Postgate, J. (1981) In Current Perspectives in Nitrogen Fixation (A. H. Gibson, W. E. Newton, eds.) Canberra: Australian Academy of Science, pp. 146-156; and Reidel, G. E., Ausubel, F. M. and F. M. Cannon (1979) Proc. Nat. Acad. Sci. U.S.A. 76:2866-2870). Three of these genes, nifH, nifD and nifK encode the structural proteins of the nitrogenase enzyme complex (viz. the Fe-protein subunit and the α- and β-subunits of the Mo-Fe protein respectively). These genes are linked on the same operon in K. pneumoniae and are transcribed from a promoter adjacent to the nifH gene. The remainder of the nitrogen fixation genes contain information required for bacterial attachment, root hair curling, initiation and development of nodules and establishment of symbiotic relationships. In addition, regulatory sequences such as promoters, operators, attenuators, and ribosome binding sites are found adjacent to the coding regions. These regulatory sequences control the expression of the structural genes, i.e., the coding sequences downstream in the 3'-direction of the DNA reading strand.

The discovery and study of plasmids, restriction enzymes, ligases and other enzymes involved in DNA synthesis has led to the rapidly developing field of genetic engineering. Use of these techniques has made it possible to transfer DNA across species boundaries, either from eukaryotic to prokaryotic

organisms or vice versa. Alternatively, it has been possible to synthesize nucleotide sequences and to incorporate these synthetic sequences into living organisms where they have been expressed. For example, expression in E. coli has been obtained with DNA sequences coding for mouse dihydrofolate reductase (Chang, A. C. Y., Nunberg, J. H., Kaufman, R. K., Ehrlich, H. A., Schimke, R. T. and Cohen, S. N. (1978) Nature 275:617-624) and for hepatitis B virus antigen (Burrell, C. J., Mackay, P., Greenaway, P. J., Hofschneider, P. H. and K. Murray (1979) Nature 279:43-47). Two mammal hormones have also been produced in bacteria by use of synthetic DNA (Itakura, K., Hirose, T., Crea, R., Riggs, A. D., Heynecker, H. L., Bolivar, F., and H. W. Boyer (1977) Science 198:1056; and Goeddel, D. B., Kleid, D. G., Bolivar, F., Heynecker, H. L., Yansura, D. G., Crea, R., Hirose, T., Kraszewski, A., Itakura, K. and A. D. Riggs (1979) Proc. Nat. Acad. Sci. U.S.A. 76:106).

The practical application of DNA recombination requires the success of a number of different features. First, it must be possible to recognize the DNA fragment coding for the compound of interest and it must be possible to isolate that DNA fragment. Second, it is necessary to understand the mechanisms which control the expression of the information on that DNA fragment and to be able to transfer that information to the control of regulatory sequences which will maximize the productive capabilities of that information. This increased productive capacity could be by rearrangement of coding information and regulatory information within the same organism or between different organisms. The organisms involved may be prokaryotic or eukaryotic. Third, the conversion of coding information into useful products, such as storage proteins and hormones, must occur in an environment where they are not subsequently degraded.

## Background of the Invention

In bacteria of the genus Rhizobium, nitrogenase synthesis is normally repressed under free-living conditions and is induced only within a complex symbiosis formed mostly with leguminous plants. R. trifolii is an example of a fast-growing Rhizobium with a narrow host range and cannot normally be induced to fix nitrogen in culture. In contrast, a Parasponia Rhizobium species has been isolated and this species is a slow-growing organism with a very broad host range capable of an effective symbiotic relationship with a

broad variety of tropical legumes as well as the non-legume Parasponia (Ulmaceae) (Trinick, M. J. (1980) J. Appl. Bacteriol. 49:39-53). Parasponia Rhizobium can be induced to fix nitrogen in culture although the level of this fixation is about 100-fold less than can be obtained from the free-living bacterium Klebsiella pneumoniae. Other slow-growing Rhizobia include the commercially significant R. japonicum, which nodulates soybeans.

The genetics of biological nitrogen fixation have been well characterized in the free-living organism Klebsiella pneumoniae. The structural genes for nitrogenase (nifH, nifD and nifK encoding the Fe-protein subunit and the α and β subunits of the Mo-Fe protein, respectively) have been mapped both genetically and physically (Kennedy, C. et al. (1981) In Current Perspectives in Nitrogen Fixation (eds. Gibson, A. H. and W. E. Newton) Australian Acad. Science, Canberra, pp. 146-156; and Reidel, G. E., Ausubel, F. M. and F. M. Cannon (1979) Proc. Nat. Acad. Sci. U.S.A. 76:2866-2870). Cloned DNA fragments carrying these sequences have been shown, by Southern blot analysis, to hybridize to homologous sequences in a wide range of nitrogen fixing organisms, including Rhizobium (Ruvkun, G. B. and F. M. Ausubel (1980) Proc. Nat. Acad. Sci. U.S.A. 77:191-195).

In spite of the ecological diversity of nitrogen fixing organisms, the physiological structure of the nitrogenase enzyme complex appears to be very conserved. In all cases where the enzyme complex has been purified, two proteins are present. The larger protein (dinitrogenase) contains molybdenum, iron and acid-labile sulfur, and carries the binding site for nitrogen and contains two subunit proteins α- and β-coded by the nifD and nifK genes respectively. The smaller protein (dinitrogenase reductase) contains iron and acid-labile sulfur, and is required for the reduction of the dinitrogenase and for the binding of MgATP used in this reduction. The dinitrogenase reductase is coded by the nifH gene. Chemical and spectral analyses of the purified protein components support a conservation of protein structure between organisms (Scott, K. F., Rolfe, B. G. and J. Shine (1981) J. Mol. Appl. Genet. 1:71-81). In some cases the structures are sufficiently similar to allow formation of active hybrid enzymes between purified components, e.g., Azotobacter vinelandii and Klebsiella pneumoniae (Eady, R. R. and B. E. Smith (1979) In: A treatise on dinitrogen fixation I, II, eds. Hardy, R. W., Bottomley, F. and R. C. Burns, New York, Wiley Press pp. 399-490). Not

surprisingly, therefore, the region of the nif operon coding for nifH and nifD shows homology at the nucleic acid sequence level with the corresponding sequences in at least 19 other bacterial strains (Ruvkun, G. B. and F. M. Ausubel (1980) Proc. Nat. Acad. Sci. U.S.A. 77:191-195). Although this conservation of structure is generally true, significant differences between nitrogenases from different organisms also exist as can be shown by variable stability following purification and by the fact that active hybrid complexes do not form in all cases (Eady, R. R. and B. E. Smith (1979) supra).

A DNA fragment carrying the Klebsiella pneumoniae nifK, nifD and nifH genes has been isolated from the $nif^-$ strain UNF841(Tn5::nifK) and cloned into the Escherichia coli plasmid pBR325. The nucleotide sequences of the nifH gene and of 622 nucleotides of the nifD gene were determined (Scott, K. F., Rolfe, B. G. and J. Shine (1981) supra). In addition, the DNA sequence of the nifH gene from Anabaena 7120 has been determined (Mevarech, M., Rice, D. and R. Haselkorn (1980) Proc. Nat. Acad. Sci. U.S.A. 77:6476-6480). A comparison of the two sequences demonstrates two interesting features: (1) There is very little homology (31%) between the two sequences at the nucleotide sequence level although a few stretches (up to 25bp) are conserved, accounting for the observed interspecies homology of the nif genes (Ruvkun, G. B. and F. M. Ausubel (1980) supra); (2) In general, the promoter regions show very little sequence homology with the exception of a short region likely to be involved in common functions, e.g., RNA polymerase recognition.

In contrast, a comparison of the amino acid sequences of the dinitrogenase reductase and of the first 207 amino acids of the α-subunit of dinitrogenase of the two species and of another species show a much greater conservatism. The three species used in this comparison are Klebsiella pneumoniae (Kp); Anabaena 7120 (Ab); and Clostridium pasteurianum (Cp) (Tanaka, M., Haniu, M., Yasunobu, T. and L. Mortenson (1977) J. Biol. Chem. 252:7093-7100). The Kp and Cp proteins share 67% amino acid sequence homology, Kp and Ab proteins share 71% homology, and the Cp and Ab proteins share 63%. This amino acid sequence homology is not spread evenly throughout the protein. Some regions are virtually identical--90% to 95% homology), while other regions are only weakly conserved (30-35% homology). The structural conservation appears to be centered around the five cysteine residues common to all three Fe proteins. These cysteine residues are believed to be ligands to the active center.

Comparison of the N-terminal amino acid sequence of the α-subunit of dinitrogenase from Cp and Kp shows very little sequence homology in this region. This is in contrast to the very high conservation of amino acid sequence seen in the amino terminal region of the Fe protein. What little homology exists between Cp and Kp α-subunits is confined to regions around cysteine residues, as in the Fe proteins. These homologous regions are thought to be involved in the catalytic functions of the nitrogenase enzyme complex. Therefore, this structural conservatism is thought not to be the result of recent evolution and dispersal of the nif genes (Postgate, J. R. (1974) Sym. Soc. Gen. Microbiol. 24:263-292) but, rather, is postulated to be related to a conservation of function.

The isolation of some of the genes involved in symbiotic nitrogen fixation in R. trifolii has involved a combination of transposon-induced mutagenesis, rapid screening for in planta specific symbiotic mutants, molecular cloning of the mutant symbiotic gene sequences and subsequent isolation of the corresponding wild type DNA fragment from an R. trifolii gene bank. The presence of a wild type symbiotic gene on the cloned DNA fragment was then confirmed by introducing it into its allelic (symbiotically defective) R. trifolii mutant strain and assaying for the restoration of the symbiotic phenotype (Scott, K. F., Hughes, J. E., Gresshoff, P. M., Beringer, J. E., Rolfe, B. G. and J. Shine (1982) J. Mol. Appl. Genetics 1:315-326).

The transposon Tn5 was introduced into R. trifolii by conjugation with E. coli strain 1830. Symbiotically defective mutants were recovered by selecting for kanamycin resistant strains. These transposon induced mutants were screened once on plants to determine which of the colonies carrying the Tn5 insertions were symbiotically defective. The phenotype of recovered mutants varied from the complete loss of ability to nodulate ($nod^-$) to the production of nodules with varying morphology but inability to fix nitrogen (fix5-4). Two $nod^+$ $fix^-$ mutants were specifically reported.

DNA isolated from various Tn5-induced symbiotic mutants was digested with EcoRI cleaved pBR322 plasmid DNA. Tn5 contains no EcoRI restriction sites. These recombinant plasmids were transformed into E. coli RR1 and the cells carrying Tn5 and flanking R. trifolii sequences were selected by kanamycin resistance encoded on the transposon. The Tn5-containing recombinant plasmid DNAs were isolated, labelled in vitro with $^{32}P$, and used as hybridization

probes to identify and isolate corresponding wild type sequences. These cloned fragments were also used to examine the extent of symbiotic gene sequence homology in related species. Homologous sequences were found in some of the fast-growing strains tested, but not in the slow-growing R. japonicum.

To isolate wild type DNA sequences corresponding to the mutant sequences cloned from different kanamycin-resistant symbiotically defective mutants, several clone banks of DNA fragments from R. trifolii were generated.

To demonstrate the validity of this approach in the isolation of wild type nitrogen fixation symbiotic gene sequences, two presumptive wild types were analysed for their ability to "correct" the original Tn5-induced lesion in the R. trifolii genome. Two EcoRI fragments of 6kb and 8kb, respectively, carrying the presumptive wild type sequences were first subcloned into the broad host range conjugative plasmid RP4. These recombinant RP4 plasmids were then conjugated from E. coli RR1 into the corresponding R. trifolii mutant (i.e., the mutant used to isolate the presumptive wild type) and cells carrying the cloned symbiotic gene were assayed for their ability to carry out a normal nitrogen-fixing symbiosis on clover plants. Assuming that the cloned DNA fragment carried wild type symbiotic gene sequences, it would be expected that the original mutants would be "corrected" by one of two mechanisms. If the cloned DNA carried the complete symbiotic gene, then correction would occur by complementation and every R. trifolii cell carrying the recombinant RP4 would be capable of an effective symbiotic response. However, if the cloned DNA fragment carried only a portion of the symbiotic gene, then correction of the defect could only occur if the mutant sequences in the genome were replaced by those carried on the RP4 plasmid via homologous recombination. In this case, only a few cells, carrying the recombinant RP4, would be capable of an effective symbiotic response. Both types of responses were observed with different cloned strains. The 6kb isolate appeared to contain a complete symbiotic gene; however, the larger, 8kb isolate did not contain all of the information necessary to overcome the symbiotic defect of the Tn5-induced mutant clone to which it hybridized.

In many cloning projects, only one of the two DNA strands is required initially. Many techniques have been used including poly(UG)-CsCl gradients (Szybalski, W., Kubinski, H., Hradecna, C. and W. C. Summers (1971) Methods Enzymol., Grossman, L., and Moldave, K., eds. Vol.21D Academic Press, New York

pp. 383-413), polyacrylamide gels (Maxam, A. and W. Gilbert (1977) Proc. Nat. Acad. Sci. U.S.A. 74:560-564), and exonuclease treatment (Smith, A. J. H. (1979) Nucl. Acids. Res. 6:831-848). An alternative biological approach has been developed using the bacteriophage M13. The replicative form of this phage DNA is a circular double stranded molecule; it can be isolated from infected cells and used to clone DNA fragments after which it can be reintroduced into *Escherichia coli* cells by transfection. M13 phage particles each containing a circular single stranded DNA molecule are extruded from infected cells. Large amounts of single stranded DNA containing a cloned fragment (5-10μg phage DNA/ml bacterial culture can be easily and quickly recovered (Messing, J., Gronenborn, B., Muller-Hill, B., and P. H. Hofschneider (1977) Proc. Nat. Acad. Sci. U.S.A. 74:3642-3646). The cloning of DNA fragments into the replicative form of M13 has been facilitated by a series of improvements which led initially to the M13mp7 cloning vehicle (Messing, J., Crea, R. and P. H. Seeburg (1981) Nucleic Acids Res. 9:309-321). A fragment of the *E. coli* lac operon (the promoter and N-terminus of the β-galactosidase gene) was inserted into the M13 genome. A small segment of DNA containing a number of restriction cleavage sites was synthesized and inserted into the structural region of the β-galactosidase fragment. The M13mp7 DNA remains infective and the modified *lac* gene can still encode the synthesis of a functional β-galactosidase α-peptide.

The synthesized DNA fragment contains two sites each for the *Eco*RI, *Bam*HI, *Sal*I, *Acc*I and *Hinc*II restriction enzymes arranged symmetrically around a centrally located *Pst*I site. Therefore, by chance, either strand of a cloned restriction fragment can become part of the viral(+) strand depending on the orientation of the cloned fragment relative to the M13 genome after ligation. Insertion of a DNA fragment into one of these restriction sites can be readily monitored because the α-peptide will be non-functional, so that there will be no β-galactosidase activity.

Following M13mp7, two new single stranded DNA bacteriophage vectors M13mp8 and M13mp9, have been constructed (Messing, J. and J. Vieria (1982) Gene 19:269-276). The nucleotide sequence of M13mp7 has been modified to contain only one each of the restriction sites (instead of two) and single restriction sites for *Hind*III, *Sma*I and *Xma*I have been added. Thus the restriction sites are *Eco*RI-*Sma*I-*Xma*I-*Bam*HI-*Sal*I-*Acc*I-*Hinc*II-*Pst*I-*Hind*III.

These restriction sites have opposite orientations in M13mp8 and M13mp9. DNA fragments whose ends correspond to two of these restriction sites can be "force cloned" to one or the other of these two M13 cloning vehicles which have also been "cut" by the same pair of restriction enzymes. Thus a DNA fragment can be directly oriented by forced cloning. This procedure guarantees that each strand of the cloned fragment will become the (+) strand in one or the other of the clones and will be extruded as single stranded DNA in phage particles.

Restriction endonuclease cleavage fragments with non-complementing ends are seldom joined in a ligation. DNA cleaved by two different restriction endonucleases therefore cannot be circularized nor joined to another fragment produced by the same "two different restriction endonucleases" in both orientations. The result is that a recombinant molecule is formed during the ligation reaction with a defined order of the two fragments. Since the orientation of a cloned DNA fragment in the replicative form of M13 vectors determines which of the two DNA strands is going to be the viral strand, the use of M13mp8 or M13mp9 allows the direct preparation of one of the two DNA strands by cloning.

## SUMMARY OF THE INVENTION

A recombinant plasmid is disclosed, wherein there is a wide host range vector containing an inserted fragment of DNA including a regulatory region of a nitrogenase complex gene of a Rhizobium species and a foreign structural gene under the control of the regulatory region. Since the regulatory region and the foreign genes are carried on plasmids which can be lost from naturally-occurring Rhizobium species, a method for transferring these genes from a vector to the bacterial chromosome is also described. Novel Rhizobium strains are thereby generated, having a chromosomally integrated composite gene including a nitrogenase gene regulatory region and a foreign gene. Also disclosed are novel Rhizobium strains wherein one or more nitrogenase genes of the same or different Rhizobium species is integrated into the chromosome.

## DETAILED DESCRIPTION OF THE INVENTION

The invention is based in part on the isolation and characterization of the regulatory regions controlling the nitrogen fixation (nif) genes of

Rhizobium strains. A regulatory region can be combined with a structural gene isolated from an extraneous source organism ("foreign gene" herein) and combined in a plasmid to provide a novel plasmid bearing the foreign gene expressible under control of the nif gene regulatory region, and to provide a novel microorganism transformed by the novel plasmid. Alternatively, the composite gene, including the foreign structural gene and nif gene regulatory region, can be integrated with the chromosome of a host bacterial strain in order to maximize the stability of the trait conferred by the composite gene. Furthermore, a novel Rhizobium strain can be constructed in which a nif regulatory region together with the structural gene or genes it normally controls is integrated with the host chromosome to enhance stability of the ability to fix nitrogen.

The novel plasmids disclosed herein are useful for amplifying the quantities of composite genes, for transferring such genes to selected bacterial hosts, for generating new host bacterial strains and as intermediates for the construction of other plasmids having one or more of the foregoing uses. The bacterial strains of the present invention are useful for expressing the composite gene, under certain conditions, to provide a useful product, to confer an advantageous property to a plant or to improve the rate, quality or efficiency of the nitrogen fixation process. In particular, the properties of the novel strains are manifested within root nodules formed by novel Rhizobium strains of the invention in symbiotic combination with a host plant. Depending upon the gene chosen for expression in the nodule, the nodule then serves as a production source for a protein coded by the gene. Examples of proteins which can be expressed in root nodules include the insect-toxic protein of Bacillus thuringiensis (Wong, H. C., et al., J. Biol. Chem. 258, 1960 (1983), the hydrogenase found in some but not all Rhizobium strains (Cantrell, et al., Proc. Nat. Acad. Sci. USA 80, 181 (1983), metallothionein (Karin, M. and Richards, R. I., Nucl. Acids Res. 10, 3165 (1982) and prolactin (Cooke, N., et al. (1981) J. Biol. Chem. 256:4007-4016). The foregoing list is not intended as limiting, but merely as exemplary of the broad range of possibilities for synthesis of proteins in root and stem nodules of plants. In general the invention makes it possible to produce any protein that may be of use, either as a product extracted from the nodule, as an excretion product of the nodule, conferring an advantage for the host plant, or as a functional protein within the nodule itself, improving

the effectiveness of the symbiotic interaction. In addition to the proteins disclosed herein, others will be apparent to those of ordinary skill in the art, taking advantage of the known or subsequently discovered properties of root nodules and of specific proteins. A major advantage conferred by gene expression under control of a nif regulatory region in root nodules is derived from the inventors' recognition that such expression is regulated in a similar manner as the expression of the nif genes themselves. The foreign gene is only minimally expressed, if at all, in the free-living bacteria. However, the gene is maximally expressed within the root nodule. Furthermore, because of the specific nature of the host-bacterium symbiosis, gene expression occurs only in the selected plant species recognized by the modified bacterial strain. These properties consequently ensure, first, that the foreign gene expression provides maximum local effect of the expression product, and second, that environmental side effects are limited since gene expression can be confined to the nodular tissue of the selected crop or plant variety.

Despite the fact that many structural genes of Rhizobium nif region hybridize with previously isolated nif gene segments, as described, supra, some Rhizobium nif regions do not hybridize, as shown by Scott, et al., supra. Furthermore, the regulatory regions so far identified are substantially different from one another. In fact, as discovered in the course of the experiments leading to the present invention, the organization of the nifH, D and K genes differs within the Rhizobium species in a manner difficult to generalize at present. Many species have a single regulatory region apparently controlling the H, D and K genes. However, as disclosed herein, the Parasponia Rhizobium has a separate, distinctive region regulating expression of nifH, and this gene maps at a different genetic locus from nifD or K. As a consequence of the results herein disclosed, it now appears that the regulatory regions (promoters) of the nif genes of Rhizobia are individually distinctive, and variable in number and genetic locus, from strain to strain. The techniques disclosed herein provide the first systematic means for isolating and genetically manipulating such regulatory regions for useful purposes.

Cloned DNA fragments of the nifH, nifD and nifK genes of the free living organism Klebsiella pneumoniae were used to identify and isolate the corresponding symbiotic genes of Rhizobium trifolii ANU329 and of Parasponia

Rhizobium species ANU289. These three genes (nifH, nifD and nifK) constitute the nitrogenase complex. They may all be closely linked or they may be unlinked. In particular, a recombinant plasmid (pKnif-2) carrying K. pneumoniae DNA and coding for the entire nifH gene and the N-terminal 207 amino acids of nifD gene has been cloned and used as a hybridization probe. (Scott, K. F., Rolfe, B. G. and J. Shine (1981) J. Mol. Appl. Genet. 1:71-81).

To isolate the nitrogenase complex structural genes from R. trifolii, a gene bank of R. trifolii ANU329 DNA was constructed by partial cleavage of genomic DNA with Sau3A and ligation into BamHI-cleaved DNA isolated from the phage vector λ-Charon 28 (Scott, K. F., Hughes, J. E., Gresshoff, P. M., Beringer, J. E., Rolfe, B. G. and J. Shine (1982) J. Mol. Appl. Genet. 1:315-326). This gene bank was screened by hybridisation with $^{32}P$-labelled pKnif-2 sequences. A number of positively-hybridising clones were isolated and restriction maps were constructed. A restriction endonuclease map of the R. trifolii nifH as determined from recombinant plasmid pRt329nif-3 is shown in Fig. 1a. Shaded areas indicate regions of pKnif-2 homology. Restriction sites are abbreviated as follows: R = EcoRI; B = BamHI; Bg = BglII; H = HindIII; S = SalI; and X = XhoI. The positively-hybridising clones were subcloned into the plasmid vector pBR322. A vector is defined here as a plasmid with a single replication origin which carries and replicates one or more fragments of foreign DNA. The recombinant plasmids were transformed into E. coli HB101. The resulting recombinant plasmids were extensively mapped by restriction endonuclease analysis and were the source of DNA used for direct sequence analysis.

Sequence analysis was carried out by the chemical method (Maxam, A. M. and W. Gilbert (1980) Methods in Enzymology 65:499-560) from defined restriction sites and by a method based on the generation of a series of deletions with the double stranded exonuclease Bal31 (Legerski, R. J., Hodnett, J. L. and H. B. Gray (1978) Nucleic Acids Res. 5:1445-1464) followed by subsequent cloning of these deleted fragments into the phage vector M13mp9 (Vieira, J. and J. Messing (1982) Gene 19:259-268) and sequence analysis by the chain termination method (Sanger, F., Nicklen, S. and Coulson, A. R. (1977) Proc. Nat. Acad. Sci. U.S.A. 74:5463-5467). To illustrate the procedures, a series of overlapping M13 clones of known orientation from

recombinant plasmid pRt329nif-2 was constructed (Fig. 1b) enabling the rapid sequence analysis of some 1500 base pairs (bp) of nitrogenase complex-specific R. trifolii DNA. The shaded region indicates the location of the nifH and nifD gene sequences. Restriction sites are abbreviated as for Fig. 1a. (see supra).

The DNA sequence of the R. trifolii SU329 nifH gene, 276bp preceding the gene and the N-terminal 141 codons of nifD following nifH is shown in Fig. 2. DNA sequences referred to elsewhere are underlined. Conserved cysteine residues in the nifH gene coding region are boxed. Asterisks above nifH and codons 1-60 of nifD indicate amino acid residues conserved in R. meliloti (Torok, I. and A. Kondorosi (1981) Nucleic Acids Res. 9:5711-5723). Asterisks above residues 61-141 in the nifD gene indicate identity with the known K. pneumoniae nifD sequence. The nifH coding region was assigned to an open reading frame of 297 codons based on the homology of this translation product with previously determined Fe-protein sequences (Scott, K. F., Rolfe, B. G. and J. Shine (1981) J. Mol. Appl. Genet. 1:71-81). The open reading frame is preceded by the sequence (5'-AGGA-3') analogous to the ribosome binding site sequence found in E. coli (Shine, J. and L. Dalgarno (1975) Nature 254:34-38). The same sequence precedes an open reading frame following nifH. This reading frame has been assigned as the R. trifolii nifD gene also on the basis of sequence homology of its predicted translation product to the partial amino acid sequence analysis of the Mo-Fe protein subunit α from Clostridium pasteurianum (Hase, T., Nakano, T., Matsubara, H. and G. Zumft (1981) J. Biochem. 90:295-298) and the predicted amino acid sequence encoded by the 5'-portion of the K. pneumoniae nifD gene (Scott, K. F., Rolfe, B. G., and J. Shine (1981) J. Mol. Appl. Genet. 1:71-81). The sequence data show clearly that the nifH and nifD genes are linked on the same operon in R. trifoliii as is the case in K. pneumoniae, R. meliloti (Ruvkun, G. B., Sunderasan, V. and F. M. Ausubel (1982) Cell 29:551-559) and in R. japonicum strain 110 (Henneke, H. (1981) Nature 291:354-355). This is in contrast to the organization of the nifH and nifD genes seen in the non-legume symbiont Parasponia Rhizobium sp ANU289 (see infra).

The DNA sequence of R. trifolii 5' to the nifH gene (i.e., the fragment of DNA controlling expression of nifH structural genes, Fig. 2) shares very little homology to the corresponding sequences of other Rhizobium species

(Torok, I. and A. Kondorosi (1981) Nucleic Acids Res. 9:5711-5723) or Parasponia Rhizobium sp. ANU289 (see infra).

The sequence of the R. trifolii Fe-protein can be predicted from the DNA sequence of the nifH gene (Fig. 2). The amino acid sequence is strongly conserved when compared to C. pasteurianum (65% homology) and to K. pneumoniae, Anabaena 7120 and Azotobacter vinelandii (70% homology). The homology between the amino acid sequences of R. trifolii and the closely related legume symbiont R. meliloti is even greater, i.e., 90%.

The sequence of the N-terminal 141 amino acids from the α-subunit of the R. trifolii Mo-Fe protein can also be predicted from the sequence of the nifD gene (Fig. 2). The region of the protein from residue 61-121 shows considerable sequence homology (81%) with the K. pnuemoniae nifD α-subunit. In contrast the same comparison for residues 1-60 shows only weak conservation (10-15%). It appears, therefore, that as in the nifH gene products, the conserved amino acids occur in specific regions of the protein.

In the present invention, in addition to the isolation and characterization of the nifH and nifD genes of Rhizobium trifolii and of the DNA sequences regulating their function, the nifH and nifD genes of a slow growing, broad host range Parasponia Rhizobium sp. ANU289 have also been isolated and partly sequenced. In contrast to previously studied nitrogen fixing prokaryotes, the nifH and nifD genes are unlinked in Parasponia Rhizobium sp.

Genomic DNA isolation procedures were as described for R. trifolii. The construction of genomic libraries in the bacteriophage vector λ-Charon 28 (Liu, C. P., Tucker, R. W., Mushinski, J. F. and F. R. Blattner (1980) Science 209:1348-1353) and procedures for screening libraries have been described (Scott, K. F., Hughes, J. E., Gresshoff, P. M., Beringer, J. E., Rolfe, B. G. and J. Shine (1982) J. Mol. Appl. Genet. 1:315-326). The cloned DNA fragments of the nif genes of Klebsiella pneumoniae (used to probe for nitrogenase complex genes of Rhizobium) were again used to probe for nitrogenase complex genes of Parasponia Rhizobium sp. It was discovered that several positively-hybridising recombinants could be obtained and the restriction map of one clone (λPR289nif-1) is shown (Fig. 3a). Shaded areas indicate regions of pKnif-2 homology. The restriction sites are abbreviated as follows: R = EcoRI; B = BamHI; H = HindIII; Bg = BglII; S = SalI; X = XhoI; and P = PstI.

Only those PstI sites used in the cloning experiments are shown. The restriction map of this phage clone was shown to be identical to that of the corresponding region in the Parasponia Rhizobium sp. genome. This was determined by hybridization of $^{32}P$-labelled sub-fragments of λPR289nif-1 to restriction digests of ANU289 DNA. A 4.5 kilobase PstI fragment homologous to the K. pneumoniae nifH-specific probe was sub-cloned into the plasmid vector pBR322 and the resultant recombinant clone (pBR289nif-2) was extensively mapped by restriction endonuclease analysis (Fig. 3b). The shaded area indicates the location of the nifH gene. This clone was used as a template for the direct sequence analysis of the nifH gene.

The entire sequence of the Parasponia Rhizobium DNA including its nifH coding region and associated regulatory region has been determined (Fig. 4). DNA sequences referred to elsewhere in this invention are underlined. Conserved cysteine residues in the nifH coding region are boxed. Asterisks above the nifH coding sequences indicate amino acid residues which are conserved in all nitrogen-fixing organisms examined to date. The arrow indicates the 5' end of the nifH transcript. There is a translation initiation codon 572 base pairs (bp) from the PstI site at the 5' end of the sequence followed by an open reading frame of 882bp. The amino acid sequence predicted from the nucleotide sequence of this reading frame is homologous to that of all other nifH genes so far determined. The N-terminal methionine of this nifH gene is preceded by a purine-rich region (5'-GGAG-3') which is identified as a ribosome binding site for the initiation of translation.

The 5'-end of the nifH m-RNA transcript was mapped by an S1-nuclease procedure (Sharp, P. A., Berk, A. J. and S. M. Berget (1980) In Methods of Enzymol. 65, (L. Grossman and K. Moldave, eds.) New York, Academic Press, pp.750-758) using polyA$^-$ RNA isolated from Siratro (Macroptilium atropurpureum) nodules inoculated with ANU289. The nifH coding sequence is preceded by a long leader sequence of 155bp (Fig 4). On the basis of the S1-mapping analysis, presumptive RNA polymerase recognition sequences are assigned (Fig. 4). It is clear that the sequences in the -10 region (5'-ATTGCT-3') and -35 region (5'-TAAGCG-3') of the gene are not homologous to the consensus RNA polymerase recognition sequences preceding E. coli operons (TATAAT and TTGACA, respectively) (Siebenlist, U., Simpson, R. B. and Gilbert, W. (1980) Cell 20:269-281). More significantly the promoter sequences share

very little homology with those assigned for the Anabaena transcript (Haselkorn, R., Curtis, S. E., Fisher, R., Mazur, B. J., Mevarech, M., Rice, D., Nagaraja, R., Robinson, S. J. and R. Tuli (1982) In Cyanobacteria: Cell Differentiation and Function G. C. Papageorgiou, L. Packer, eds.) or for Rhizobium transcripts (Fig. 2). Significantly the primary sequences of the regulatory regions between different nitrogen fixing organisms vary fundamentally. These differences are related to the specificity of the interaction between a plant species and a nitrogen fixing bacterial species.

In all nitrogen-fixing species examined previously the nifH gene is immediately followed by the gene coding for the α-subunit of the Mo-Fe protein, i.e., the nifD gene. The N-terminal sequences of the nifD gene from K. pneumoniae (Scott, K. F., Rolfe, B. G. and J. Shine (1981) J. Mol. Appl. Genet. 1:71-81), R. trifolii (supra) and R. meliloti (Torok, I. and Kondorosi, A. (1981) Nucleic Acids Res. 9:5711-5723) have been determined and the predicted protein sequences encoded by these nifD genes are conserved. Surprisingly, however, in Parasponia Rhizobium sp. ANU289, no sequence analogous to the Klebsiella or Rhizobium nifD genes can be found in the 591bp following the nifH gene (Fig. 4). It has now been shown that the nifH gene is separated by at least 13 kilobases from the nifD gene. Hybridization analysis of the phage clone λPR289nif-1 DNA (Fig. 3a) with a fragment of K. pneumoniae DNA encoding nifD specific sequences showed that there were no sequences homologous to the K. pneumoniae nifD probe on the 13kb of DNA following nifH. This unexpected finding was confirmed by the isolation of clones carrying nifD specific sequences from the genomic library and hybridization analysis of these clones with nifH specific sequences. Such an analysis demonstrates that nifH is not encoded on these cloned fragments. Clearly, the nifH and nifD genes are not encoded on the same operon in Parasponia Rhizobium sp. ANU289. Furthermore, as disclosed herein, the nifK gene encoding the remaining component of the nitrogenase enzyme complex (β-subunit of the Mo-Fe protein) has been mapped by hybridization analysis to be immediately to the 3'-side of nifD.

The regulatory region of the nifD gene of Parasponia Rhizobium sp. ANU289 consisting of 174bp and the coding region for the N-terminal 422 amino acids of the α-subunit protein have been determined (Fig. 5). A comparison of the amino acid sequence predicted from this coding region with the amino acid

sequence products of other nifD genes shows that the sequence is conserved. Surprisingly, the regulatory sequences to the 5' side of the methionine initiation codon bear very little resemblance to the regulatory sequences of the nitrogenase complex genes in other species, e.g., R. trifolii, or to the regulatory sequences of the nifH gene of the same species, i.e., Parasponia Rhizobium ANU289 (Fig. 6).

## EXPRESSION OF FOREIGN GENES BEHIND THE NITROGENASE COMPLEX PROMOTERS

A principle feature of the present invention is the construction of a plasmid having an inserted foreign structural gene under control of a nitrogenase complex promoter. The structural gene must be inserted in correct position and orientation with respect to the nitrogenase complex promoter in order to obtain expression of the structural gene controlled by the promoter. Position has two aspects. The first relates to which side of the promoter the structural gene is inserted. It is known that the majority of promoters control initiation of transcription and translation in one direction only along the DNA. The region of DNA lying under promoter control is said to lie "downstream" or alternatively on the 3'-side of the promoter. Therefore, to be controlled by the promoter, the correct position of plant structural gene insertion must be "downstream" from the promoter. The second aspect of position refers to the distance, in base pairs, between functional elements of the promoter, for example the transcription initiation site, and the translational start site of the structural gene. Substantial variation appears to exist with regard to this distance, from promoter to promoter. Therefore, the structural requirements in this regard are best described in functional terms. Optimum spacing can be achieved by experiments varying the length of this distance. As a first approximation, reasonable operability can be obtained when the distance between the promoter and the inserted structural gene is similar to the distance between the promoter and the gene it normally controls. Orientation refers to the directionality of the structural gene. By convention, that portion of a structural gene which ultimately codes for the amino terminus of a protein is termed the 5' end of the structural gene, while that end which codes for amino acids near the carboxyl end of a protein is termed the 3' end of the structural gene. Correct orientation of a structural gene is with the 5' end thereof proximal to the promoter. An additional requirement in the case of constructions leading to fusion protein

expression is that the insertion of the structural gene into an existing nitrogenase complex structural gene sequence must be such that the coding sequences of the two genes are in the same reading frame phase, a structural requirement which is well understood in the art.

In order to express foreign genes on the 3'-side of the nitrogenase complex regulatory sequences, it is first advantageous to construct a double stranded DNA sequence corresponding to the nifH or nifD regulatory sequences. To achieve this, synthetic DNA primer complementary to the ribosome binding site of the m-RNA and extending a few nucleotides to the side thereof is first constructed. Then the cloned nifH or nifD fragment is excised from the vector, purified and the excised nifH or nifD fragments are ligated into appropriate M13 vectors. The resultant recombinant DNA plasmids are then transformed into E. coli strains, and single colonies are propagated. Those colonies which extrude single stranded templates corresponding to the m-RNA strand are isolated. The synthetic DNA is used as a primer on these single stranded templates to generate double stranded DNA by primer extension with DNA polymerase I (Klenow fragment). This double stranded DNA will extend from the ribosome binding site to an indeterminate point within the M13 vector. Any single-stranded regions are removed by S1 nuclease treatment.

Then synthetic EcoRI linkers are ligated to the DNA fragments followed by digestion with EcoRI and that restriction endonuclease (termed endonuclease A for generality) which recognizes the restriction site at the 5' end of the nifH or nifD. The resultant DNA fragments are then cloned into an EcoRI-endonuclease A cleaved plasmid, transformed into a suitable E. coli host and amplified. The choice of plasmid is based on principles of operating convenience and location of the appropriate restriction sites, as will be understood by those of ordinary skill in the art.

Following amplification, isolation and repurification, this same plasmid is then cleaved with endonuclease A and treated with S1-nuclease or BAL-31 for a short time to produce blunt ended fragments. The plasmid is now cleaved with EcoRI and the fragment is cloned into the wide host range plasmid pRK290 which has been cleaved with SmaI-EcoRI (pRK290-nif regulatory fragment construct). Alternatively, another wide host range plasmid, pSUP204, can be used to construct the recombinant nif regulatory plasmid.

Alternatively, the DNA fragments provided with EcoRI-endonuclease A-specific ends are initially cloned into a mobilizable broad host range vector capable of replication in either E. coli or most other gram-negative bacteria, such as pSUP104 or pSUP204, described by Puhler, A. et al., supra. After amplification, the recombinant plasmid is transferred directly to the desired recipient strain.

In order to clone and express foreign genes, appropriate DNA fragments carrying these foreign genes are isolated and synthetic EcoRI linkers are ligated to the fragments. (EcoRI-foreign gene-EcoRI) These EcoRI-foreign gene-EcoRI DNA fragments are then ligated into EcoRI-cleaved vector DNA, for example, pSUP104 or pSUP204, resulting in a nif-regulated expression plasmid, pSS104 or pSS204, respectively, and transformed into Escherichia coli. After selection and amplification, the nif-regulated expression plasmid is then transferred with the aid of helper plasmids to the appropriate Rhizobium or Agrobacterium strain by mating.

The transformed Rhizobium strains are then used to infect siratro plants or other appropriate legumes which are subsequently assayed for the production of foreign mRNA and/or protein.

## INTRODUCTION OF DNA SEQUENCES INTO THE CHROMOSOME GENOME OF GRAM-NEGATIVE ORGANISMS OTHER THAN E. COLI

The nif and nod genes of R. trifolii which have been cloned and analysed, are all carried on the same large symbiotic (sym) plasmid. However, plasmids are lost rather easily from bacterial strains, leading to the loss of expression of those genes carried on the plasmids. One method of stabilizing the expression of certain genes carried on plasmids, or, for that matter, any foreign DNA segment, would be the introduction of such genes or foreign DNA segments, hereinafter termed "introduced DNA", into the chromosome of the host bacteria. Such a system employs a "suicide vector" and, preferably, a transposon.

Suicide vectors are plasmid molecules which replicate stably in one bacterial host (in this case, Escherichia coli) but fail to replicate in a different bacterial species (e.g., Rhizobium trifolii).

Transposons are genetic elements which are able to move (translocate) from one location to another in DNA. The translocation process is mediated by gene products encoded on the transposon and is dependent upon the integrity of repeated sequences (directly or indirectly repeated) located at each end of the transposon. Transposons generally carry a gene (or genes) encoding resistance to one (or more) antibiotics. The transposon and the suicide vector are linearized and religated into a single recombinant DNA molecule.

The general method of transferring introduced DNA segments to the chromosome of a gram-negative bacterial strain other than E. coli is outlined here. The introduced DNA fragments to be introduced can be generated in a number of ways: (a) by restriction with site specific restriction endonucleases, (b) by partial or complete digestion with restriction endonucleases which generate DNA fragments having blunt ends, (c) by digestion of DNA with the enzyme DNAaseI in the presence of $Mn^{2+}$ ions thus generating random fragments which are generally blunt-ended, or (d) by shearing the DNA into large fragments.

In the preferred method, the suicide vector carrying a transposon with an antibiotic resistance gene is linearized and the appropriate fragment of introduced DNA is ligated into a "co-integrated recombinant molecule." The fragment of DNA is inserted into a restriction endonuclease site within the transposon such that the insertion does not disrupt normal transposition nor expression of the drug resistance marker. This ligated DNA is then transformed into an E. coli strain in which it can be amplified and mobilized for transfer into other gram-negative bacteria.

Introduction of the cloned introduced DNA fragment from this E. coli strain into the chromosome of any gram-negative bacterium, e.g., Rhizobium trifolii, is most conveniently achieved by the process of bacterial conjugation. The E. coli strain carrying the suicide vector which contains an antibiotic resistance gene, is mixed with cells of the antibiotic sensitive gram-negative strain on the surface of a nutrient agar plate. The plate is incubated for a period (4-16 hr.) at the optimum temperature of the gram-negative strain and during this time, cells of each bacterial species come into physical contact (conjugation) and the suicide vector is transferred from the donor E. coli to the recipient gram-negative strain. The cell mixture is washed off the plate and spread on an agar plate which is selective for the

antibiotic resistance. It is preferred to include selection means that select against growth of the E. coli parent strain once the conjugation is completed.

Since the suicide vector containing the introduced fragment of DNA cannot be amplified autonomously in the recipient gram-negative strain, a transfer of genetic material to the bacterial chromosome can occur in one of three ways: (a) If a fragment of the recipient gram-negative bacterial chromosome (BC) has been previously inserted into the suicide vector (SV) thus creating a region of homology between the suicide vector and the recipient gram-negative bacterial chromosome, then a single reciprocal recombination will result in the incorporation of the entire "co-integrated recombinant molecule" into the chromosome of the recipient gram-negative bacterial chromosome (Fig. 7). The boxed area indicates the position of insertion of the introduced gene fragment and $D^R$ indicates the position of the antibiotic resistance gene. (b) If a fragment of the recipient gram-negative bacterial chromosome has been previously inserted into the suicide vector thus creating a region of homology between the suicide vector and the recipient gram-negative bacterial chromosome and then an introduced DNA fragment and a drug resistance gene are inserted into this region of homology, a double reciprocal recombination event will incorporate only the introduced DNA fragment and the drug resistance gene into the chromosome of the recipient gram-negative bacterial strain (Fig. 8). Such recombination is site-specific, the chromosomal location being determined by the fragment of chromosomal DNA carried on the suicide vector. The components of the figure are labelled as in Fig. 7. (c) In the preferred method, the transposon containing an introduced DNA fragment and an antibiotic resistance gene may be transposed into the bacterial chromosome of the recipient gram negative bacterial strain (Fig. 9). The components of the figure are labelled as in Fig. 7. In addition, Tn refers to a transposon used to transpose the inserted DNA into the bacterial chromosome. Selection for the antibiotic resistance ensures maintenance of the inserted DNA.

Example 1: Isolation of DNA

Genomic DNA was isolated from individual bacterial colonies as previously described (Scott, K. F., Rolfe, B. G. and J. Shine (1981) J. Mol. Appl. Genet. 1:71-81). Extraction of DNA from liquid cultures was done by the same procedure except that the cell pellet from 5ml culture was resuspended in 1ml 25% sucrose in TE (10mM Tris-HCl pH 8.0 1mM EDTA) and incubated for 30 minutes at room temperature with lysozyme (1mg/ml) and EDTA (50mM). The suspension was diluted to 20ml with TE and cells were lysed by the addition of SDS (to 0.4% w/v) and Pronase (100μg/ml) with incubation at 37°C for two hours. The lysate was extracted twice with phenol/chloroform (equal volume 1:1) and dialysed against two changes of 0.3M NaCl in TE. DNA was precipitated with 2.5 volumes of ethanol at -20°C.

For isolation of plasmid DNA, cultures were grown in L-broth and amplified by the addition of spectinomycin (250μg/ml). Plasmid DNA was isolated by a polyoxyethylene ether-deoxycholate lysis procedure (Watson, J., Schmidt, L. and N. Willets (1980) Plasmid 4:175-183). Cells from one liter cultures were harvested by centrifugation and resuspended in 10ml cold 25% (w/v) sucrose in TE. After addition of lysozyme (3mg/ml) and EDTA (200mM), the cells were lysed by the rapid addition of 15ml 1% (v/v) polyoxyethylene ether 58, 0.4% (w/v) sodium deoxycholate in TE and incubation at 4°C for 10-20 minutes. The lysate was centrifuged at 17,000 rpm for 40 minutes to pellet cellular debris. DNA was precipitated from the supernatant by the addition of 3% (w/v) NaCl and 1/4 volume 50% (w/v) polyethylene glycol 6000 with incubation on ice for at least 2 hours. After centrifugation at 5000 rpm for two minutes, the DNA pellet was resuspended in 5ml 50mM NaCl in TE before addition of 8g cesium chloride and 0.6ml ethidium bromide (10mg/ml) and incubation on ice for 30 minutes. Excess polyethylene glycol was removed by centrifugation at 10,000 rpm at 4°C for 30 minutes. The density of the supernatant was adjusted to 1.59-1.61 g/ml by the addition of 1.5ml 50mM NaCl in TE. Plasmid DNA was banded by centrifugation at 100,000g for 40 hours at 18°C.

Example 2: Molecular cloning and hybridization procedures

The construction of genomic libraries in the bacteriophage vector λ-Charon 28 (Liu, C. P., Tucker, P. W., Mushinski, J. F. and F. R. Blattner

(1980) Science 209:1348-1353) and procedures used for screening libraries have been described previously (Scott, K. F., Hughes, J. E., Gresshoff, P. M., Beringer, J. E., Rolfe, B. G. and J. Shine (1982) J. Mol. Appl. Genet. 1:315-326).

Hybridization probes were prepared by primed synthesis with DNA polymerase I (Klenow fragment) using denatured random calf thymus DNA primers. Linearized plasmid DNA (100mg) was heat-denatured by boiling for 2 minutes with 100μg random primers (8-12 nucleotide fraction of DNAase I-treated calf thymus DNA) in 20μl and cooled on ice for 30 seconds. Denatured DNA was incubated for 30 minutes at 37°C with 1 unit DNA polymerase I (Klenow fragment) in 10mM Tris-HCl pH 7.4, 8mM MgCl425, 10mM β-mercaptoethanol, 600μM each of dGTP, dATP, and dTTP and 30uCi $\alpha$-$^{32}$P-dCTP (> 7000 Ci/mmol, Amersham). The reaction was stopped by phenol/chloroform extraction and the aqueous phase passed over a Sephadex G-50 column to remove unincorporated radioactivity. Peak fractions (specific activity $10^7$ - $10^8$ cpm/μg DNA) were precipitated by the addition of *E. coli* tRNA (20μg) and 2.5 vol. ethanol at -20°C.

DNA was transferred from agarose gels and immobilized on nitrocellulose sheets by depurination, denaturation and blotting as described (Southern, E. M. (1975) J. Mol. Biol. 98:503-517).

Example 3: DNA sequencing

Sequence data was obtained by the use of both the chemical cleavage method (Maxam, A. M. and Gilbert, W. (1980) In Methods in Enzymol., 65, L. Grossman and K. Moldave, eds. (New York, Academic Press, pp. 499-560) and the chain termination method (Sanger, F., Nicklen, S., and A. R. Coulson (1977) Proc. Nat. Acad. Sci. U.S.A. 74:5463-5467). For the latter procedure, template was generated by the construction of a series of defined deletions with the exonuclease Bal31 followed by cloning into the bacteriophage vector M13mp8 as follows. Plasmid DNA (5μg) was linearized with the endonuclease *Xho*I, digested with Bal31 (20 units) in 20mM Tris-HCl pH8.1, 12mM $CaCl_2$, 600mM NaCl and 1mM EDTA at 31°C. Samples (10μl) were taken at one minute time intervals and the digestion stopped by phenol-chloroform extraction and ethanol precipitation. The Bal31-digested DNA was then cleaved with *Eco*RI, ligated into *Eco*RI-*Hinc*II cleaved M13mp8 DNA and transformed into *E. coli*

JM103 cells (Scott, K. F., Rolfe, B. G., and J. Shine (1981) J. Mol. Appl. Genet. 1:71-81). Template DNA was isolated and sequenced.

Example 4: Molecular cloning and DNA sequence of the nifH (Fe-protein) gene from Rhizobium trifolii ANU329

Genomic DNA was isolated from *R. trifolii* ANU329 and partially cleaved with the restriction endonuclease *Sau*3A. The resulting DNA fragments were ligated into *Bam*HI cleaved λ-Charon 28 DNA and the phage DNA was packaged *in vitro* to generate an ANU329 library. This library was screened by hybridization with the 750bp *nif*H specific fragment from pK*nif*-2 (Scott, K. F., *et al*. (1981) *supra*). DNA was prepared from the positively hybridising recombinant (pRt329*nif*-3) and then cleaved with *Hind*III. The resultant *Hind*III restriction fragments were ligated into *Hind*III cleaved pBR322 and transformed into *E. coli* RR1. Recombinants were selected by hybridization to pK*nif*-2 sequences. DNA was prepared from the recombinant plasmid (pRt329*nif*-2) and sequenced by chemical and chain termination methods.

Example 5: Molecular cloning and DNA sequence of the nifH (Fe-protein) gene from Parasponia Rhizobium sp. ANU289

Genomic DNA was isolated from *Parasponia Rhizobium* sp. ANU289 and partially cleaved with the restriction endonuclease *Sau*3A. The resulting DNA fragments were ligated into *Bam*HI cleaved λ-Charon 28 DNA and the phage DNA was packaged *in vitro* to generate an ANU289 library. This library was screened by hybridization with the 750bp *nif*H specific fragment from pK*nif*-2 (Scott, K. F., *et al*. (1981) *supra*). DNA was prepared from the positively-hybridizing recombinant (PR289 *nif*-1) and cleaved with *Pst*I. The resultant *Pst*I restriction fragments were then ligated into *Pst*I cleaved pBR322 and transformed into *E. coli* RR1. Recombinants were selected by hybridization to pK*nif*-2 sequences. DNA was prepared from the recombinant plasmid (pPR289-*nif*-2) (Fig. 3) and sequenced by chemical and chain termination methods. A *Pst*I-*Bam*HI fragment was obtained by cleaving pBR289-*nif*-2 with the restriction endonucleases *Pst*I and *Bam*HI and then purifying the fragment (see Fig. 3).

Example 6: Expression of foreign genes under the control of a nif regulatory DNA region. Method I

Construct a synthetic DNA primer which is complementary to the ribosome binding site of the ANU289 nifH gene (5'-CTCCATCAACCG-3'). Strain ANU289 is a streptomycin resistant strain of Parasponia Rhizobium species derived from strain CP283. The nifH specific PstI-BamHI fragment which includes the nifH regulatory region (nifH-R.R.) (see Example 5) is then subcloned into M13mp9, transformed into E. coli JM103 (Fig. 10) and incubated in 0.1M salt at 30°C. The cloned fragment is amplified and single stranded templates corresponding to the mRNA strand of ANU289 nifH are packaged and extruded into the media. The single stranded templates (ca. 1μg) are recovered from the supernatant following centrifugation of the bacterial host. A 10-fold excess of the synthetic DNA primer in the presence of the four deoxynucleotide triphosphates (one of which is radioactive) and DNA polymerase I (Klenow fragment) is now used as a primer on this nifH template to generate double stranded DNA (Fig. 11). The mixture is incubated for 15 minutes at 37°C during which time more than 500 nucleotides are incorporated into the complementary strand. The remaining single stranded DNA is then removed by digestion with S1-nuclease (Fig. 11). EcoRI linkers (CGAATTCC) are then ligated to the double stranded DNA fragments followed by digestion with EcoRI and PstI (Fig. 12). The fragments are separated by agarose gel electrophoresis and the 567 base pair fragment is eluted and cloned into the wide host range plasmid pSUP204 (Fig. 13) or pSUP104 (Fig. 14), each of which has previously been restricted by the restriction enzymes PstI and EcoRI. The resulting recombinant plasmids are pSS204 and pSS104. Following transformation and amplification in a suitable E. coli host strain, e.g., SM10, cleavage with EcoRI allows the addition of any foreign structural gene or foreign DNA fragment into the linearized pSS204 or pSS104. For example, the human prolaction gene can be inserted (Cooke, N. et al. (1981) J. Biol. Chem. 256:4007-4016) or the human metallothionein gene can be inserted (Karin, M. and R. I. Richards (1982) Nucleic Acids Res. 10:3165-3173 and see Example 7) resulting in a co-integrated recombinant.

Following insertion of a foreign gene into linearized pSS204 or pSS104, the resulting co-integrated recombinant is transformed into a suitable E. coli host strain, e.g., SM10 or RR1. Subsequently the co-integrated recombinant is transferred to a Rhizobium species, e.g., Rhizobium trifolii, by bacterial

conjugation using a helper plasmid such as RP4 if necessary. The Rhizobium species carrying the co-integrated recombinant is then used to infect siratro plants and later the root nodules are assayed for the production of foreign mRNA and/or protein by standard methods known in the art.

Example 7: Insertion of the human methallothionein gene into the recombinant plasmid pRK290-nifH-R.R. Method II

The procedure followed in this example is the same as that followed in Example 6 up to the point where EcoRI linkers are ligated to the double stranded DNA fragments followed by digestion with EcoRI and PstI (Fig. 12). The resultant DNA fragments (approximately 567 base pairs) are then cloned into EcoRI-PstI cleaved pBR322. Following transformation and amplification in a suitable E. coli host strain, the recombinant plasmids are cleaved with PstI and treated with S1 nuclease for a short time to remove the 3'-overhang. The recombinants are then cleaved with EcoRI (Fig. 15) and the double stranded nif regulatory fragment is cloned into SmaI-EcoRI cleaved pRK290 DNA (Fig. 16). The resultant recombinant is thus a pRK290-nif-regulatory fragment construct (pRK290-nifH-R.R.). pRK290 is a wide host range plasmid.

The next step is to isolate DNA fragments carrying the foreign genes of interest and to ligate synthetic EcoRI linkers to these fragments. These modified fragments are then ligated into EcoRI cleaved vector DNA (i.e., the pRK290-nif regulatory fragment constructs) giving a co-integrated recombinant (pRK290-nif regulatory fragment-foreign gene) (Fig. 16) and transformed into an E. coli host strain, e.g., SM10 or RR1. The co-integrated recombinant is then transferred to a Rhizobium species by bacterial conjugation using a helper plasmid whenever necessary. The Rhizobia carrying the co-integrated recombinant are then used to infect siratro plants and later assayed for the production of foreign mRNA and/or protein by standard methods known in the art.

Example 8: Insertion of the human metallothionein gene into the recombinant plasmid pSS104

Total RNA was extracted from HeLa cells (Chirgwin, J. M., Przybyla, A. E., MacDonald, R. J. and W. J. Rutter (1979) Biochemistry 18:5294-5299) which

had been maximally induced to synthesize metallothionein by incubating in $10^{-5}$ M cadmium chloride plus $10^{-4}$M cycloheximide for eight hours before harvesting (Karin, M., Andersen, R. D. and H. R. Herschman (1981) Eur. J. Biochem. 118:527-531). Poly-A containing RNA was selected by hybridization to oligo(dT)-cellulose (Aviv, H. and P. Leder (1972) Proc. Nat. Acad. Sci. U.S.A. 69:1408-1412) and used as template for the synthesis of double stranded cDNA by sequential reverse transcriptase reactions. The hairpin bends were removed by S1 nuclease digestion and then homopolymeric dCMP tails were added to the 3'-termini of the cDNA by incubation with terminal transferase and dCTP (Chang, A. C. Y., Nunberg, J. H., Kaufman, R. J., Erlich, H. A., Schimke, R. T. and S. N. Cohen (1978) Nature 275:617-624). The double-stranded dCMP-tailed cDNA sequences were annealed to plasmid pBR322 DNA, previously linearized with restriction endonuclease PstI and tailed at the 3' ends with dGMP residues. The resulting recombinant plasmid DNA was used to transform E. coli RRI. Colonies carrying the recombinant plasmid were recognized by their $Amp^s$, $Tet^r$ phenotype. Bacterial colonies containing recombinant plasmids were grown and fixed on 0.45μ nitrocellulose filters (Grunstein, M. and D. S. Hogness (1975) Proc. Nat. Acad. Sci. U.S.A. 72:3961-3965). Duplicate filters were hybridized with $^{32}P$-labelled cDNA synthesized from poly-A containing mRNA from either induced or uninduced HeLa cells (see supra). Colonies which were judged by radioautography to give a stronger hybridization signal with induced cDNA were selected. A second test was by hybridization to a cloned mouse metallothionein-I cDNA clone (Durnam, D. M., Perrin, R., Gannon, F. and R. D. Palmiter (1980) Proc. Nat. Acad. Sci. U.S.A. 77:6511-6515). The positive clones were finally verified as human metallothionein genes by nucleic acid sequence analysis.

The human metallothionein recombinant clone is then restricted with NcoI and the overhangs are filled in to blunt ends (Maniatis, T. Jeffrey, A. and D. G. Kleid (1975) Proc. Nat. Acad. Sci. U.S.A. 72:1184-1188). EcoRI linkers are then added to these blunt ends (Fig. 17) and the fragment is inserted into pSS104 as shown above (Example 6 and Fig. 12). Those co-integrated recombinants carrying the human metallothionein gene are transformed into an E. coli strain, e.g., SM10, and subsequently transferred to a Rhizobium species by bacterial conjugation. Rhizobia carrying the co-integrated recombinant are used to infect siratro plants and the expression of the metallothionein gene in the root nodules is monitored by detection of mRNA and/or protein synthesis by standard methods known in the art.

Example 9: Insertion of the bacterial toxin gene from Bacillus thuringiensis into the recombinant plasmid pSS204

Recombinant plasmids containing inserts of the gene encoding the toxic crystal protein of B. thuringiensis are obtained using the techniques described (Wong, H. C., Schnepf, H. E., and H. R. Whiteley (1983) J. Biol. Chem. 258:1960-1967). The recombinant plasmid pES1 (ATCC Number 31995) consisting of the plasmid vector pBR322 and DNA homologous to the 30, 32 and 37 megadalton plasmids, as well as DNA homologous to linearized forms of the very large plasmids of B. thuringiensis is partially cleaved with EcoRI to give linear molecules. These partial cleavage products are further restricted by the enzyme AvaI. The digestion conditions are as recommended by the manufacturer. A probe for the toxic crystal protein gene is isolated and radioactively labelled as previously described (Wong, H. C., Schnepf, H. E. and H. R. Whiteley (1983) see supra). The restriction fragments are separated by agarose gel electrophoresis and the labelled probe is found to hybridize to one fragment of approximately 15 kilobases (kb). This fragment includes the EcoRI fragments D and F (Wong, et al., supra). The 15 kilobase fragment is then cloned into M13mp8 or M13mp9 according to standard procedure (Messing, J. and J. Vieira (1982) Gene 19:269-276) and transformed into E. coli JM103. The single stranded DNA from the extruded phage particles is purified and replicated in vitro by use of a synthetic primer (5'-TGTTATCCATGGGTTACCTCC-3') (The general method of site specific mutagenesis is described in Zoller, M. J. and M. Smith (1982) Nucleic Acids Research 10:6487-6500.) The resulting double-stranded recombinant plasmid is then tranformed back into E. coli JM103 and amplified. The amplified double-stranded plasmid DNA is purified from the E. coli JM103 cells and cleaved with the restriction endonuclease NcoI and AvaI. NcoI cleaves at the site of the synthetic primer (which is the initiation site of the toxic crystal protein gene) and AvaI cleaves at a site which is downstream from the 3'-end of the toxic crystal protein gene. The overhangs are the filled in to blunt ends (Maniatis, T., Jeffrey, A., and D. G. Kleid (1975) see supra).

Finally the pSS204 recombinant plasmid which is derived from pSUP204 (Fig. 13) is cleaved with EcoRI and the overhangs filled in to blunt ends. HindIII linkers are then added to both the B. thuringiensis toxic crystal

protein gene fragment and to the pSS204 recombinant. Following the HindIII digestion of both components, the toxic crystal protein gene and the pSS204 recombinant plasmid are ligated together to give a pSS204-*B. thuringiensis* toxic crystal protein gene co-integrate. The mixture is transformed into a suitable *E. coli* host, e.g., K802, SM10 or RR1. Plasmids are isolated from individual colonies and the orientation determined by restriction mapping. A colony containing a plasmid with the correct orientation is then conjugated to a *Rhizobium* strain and the plasmid is transferred as already described (Example 6). The production of mRNA and/or the toxic crystal protein is monitored as already described (Wong, *et al.*, *supra*).

Example 10: Introduction of DNA sequences into the genome of gram-negative organisms other than *E. coli*

This example is based on the following general principles. Two basic components are required. These are: (1) a suicide vector, and (2) a transposon.

Suicide vectors are plasmid molecules which replicate stably in one bacterial host (in this case, *Escherichia coli*) but fail to replicate in a different bacterial species (e.g., *Rhizobium trifolii*).

Transposons are genetic elements which are able to move (translocate) from one location to another in DNA. The translocation process is mediated by gene products encoded on the transposon and is dependent upon the integrity of repeated sequences (directly or indirectly repeated) located at each end of the transposon. Transposons generally carry a gene (or genes) encoding resistance to one (or more) antibiotics.

In the protocol to be outlined below, use is made of the transposon designated Tn5 and the suicide vector pSUP1011 (Simon, R., Priefer, U. and A. Puhler (1981) Proc. of Bielefeld Symposium. Springer-Verlag, West Germany) (see Fig. 18).

Transposon Tn5 is a DNA element of 5.7 kilobases (kb) in length, consisting of 1.5kb inverted repeat sequences flanking a 2.7kb central region. Encoded within one of the inverted repeats are the functions required for transposition. The central region of the transposon carries a gene conferring resistance to the antibiotic kanamycin ($Km^r$). In the middle of the

central region is a DNA sequence which is recognized by the restriction endonuclease BamHI. In the suicide vector pSUP1011, the only site recognized and cut by BamHI is that located within the Tn5 element. Experiments (Simon, R., Priefer, U. and A. Puhler (1983) Proc. of Bielefeld Symposium. Springer-Verlag, West Germany) have shown that insertion of DNA fragments into the BamHI site of Tn5 does not disrupt normal transposition nor expression of the kanamycin-resistance gene of the resultant "hybrid" transposon.

The DNA fragment to be introduced can be generated in a number of ways:

1) Complete or partial restriction with BamHI, Sau3A, MboI, etc. which generate fragments having the same, complementary, single-stranded ends.

2) Partial or complete digestion with restriction endonucleases which generate DNA fragments having blunt ends.

3) Digestion of DNA with the enzyme DNAaseI in the presence of $Mn^{++}$ ions which generates random fragments which (generally) are blunt ended.

The suicide vector (pSUP1011) DNA is treated as follows depending on the type of fragment to be cloned (above):

1) Complete restriction with endonuclease BamHI and treatment with the enzyme alkaline phosphatase.

2) Complete restriction with BamHI followed by either:

a) treatment with S1 nuclease to remove the single-stranded ends, or

b) "filling in" of the single-stranded ends by the enzyme reverse transcriptase in the presence of nucleotide triphosphates.

Each of the above treatments is followed by treatment with alkaline phosphatase.

Cloning: Vector and fragment DNA, prepared as above, are mixed and treated with the enzyme T4 DNA ligase. The ligated DNA is then transformed

(introduced) into Escherichia coli strain SM10. (This strain is capable of mobilizing ($Mob^+$) pSUP1011 derivatives (recombinant plasmids) into other gram-negative bacteria.) (Simon, R., Priefer, U. and A. Puhler (1983) Proc. of Bielefeld Symposium. Springer-Verlag, West Germany). The resultant transformants are screened by the Grunstein and Hogness colony hybridization procedure (Grunstein, M. and D. S. Hogness (1975) Proc. Nat. Acad. Sci. U.S.A. 72:3961) to detect those containing the desired cloned DNA fragment.

Introduction of the cloned DNA fragment into the genome of any gram-negative bacterium (e.g., Rhizobium trifolii) is achieved via a process called bacterial conjugation. The E. coli SM10 derivative, carrying the desired pSUP1011 recombinant, is mixed with cells of (kanamycin-sensitive) R. trifolii on the surface of a nutrient agar plate. The plate is incubated for a period (4-16 hours) at 29-30°C (optimum temperature for R. trifolii) and during this time cells of each type come into physical contact (conjugation) and the pSUP1011 derivative is transferred from E. coli to R. trifolii. The cell mixture is washed off the plate and spread on an agar plate which is selective for kanamycin-resistant R. trifolii. The resultant colonies will be derivatives of R. trifolii in which the cloned DNA fragment, within Tn5, will be inserted at some point in the genome. Selection for kanamycin resistance ensures maintenance of the inserted DNA.

At this stage it is unknown whether the DNA fragment, within Tn5, has been transferred to the chromosome of R. trifolii or to one of its several plasmids. This uncertainty can be resolved by visualization of the plasmids and the bacterial chromosome by ethidium bromide staining after horizontal agarose gel electrophoresis (Djordjevic, M. A., Zurkowski, W. and B. G. Rolfe (1982) J. Bacteriol. 151:560-568).

The following bacterial strains were deposited at the Northern Regional Research Center, U.S. Department of Agriculture, 1815 North University Street, Peoria, Illinois 61604, U.S.A.:

Strain 1 E. coli RR1/pPR289nif-2

Strain 2 E. coli RR1/pRt329nif-2

The date of deposit was June 17, 1983, and the accession numbers are: Strain 1 (NRRL-B-15446) and Strain 2 (NRRL-B-15445).

The plasmids were deposited with the American Type Culture Collection, 12301 Parklawn Drive, Rockville, Maryland 20852, U.S.A.

Plasmid strain 1 pPR289nif-2

Plasmid strain 2 pRt329nif-2

Plasmid strain 3 pSS104

The date of deposit was June 17, 1983, and the accession numbers are: Plasmid strain 1 (40073); Plasmid strain 2 (40072) and Plasmid strain 3 ( ).

CLAIMS:

1. A bacterial strain containing and replicating therein a recombinant DNA plasmid comprising;

   (a) a vector

   (b) a fragment of DNA controlling expression of a nitrogenase complex structural gene, and

   (c) a structural gene under control of said fragment of DNA.

2. A bacterial strain as recited in Claim 1 wherein said strain is a species of the genus Rhizobium.

3. A bacterial strain as recited in Claim 1 wherein said structural gene is obtained from Rhizobium species.

4. A bacterial strain as recited in Claim 1 wherein said structural gene is a foreign gene.

5. A recombinant DNA plasmid comprising;

   (a) a vector and

   (b) a fragment of DNA controlling expression of a nitrogenase complex structural gene.

6. A recombinant DNA plasmid as recited in Claim 5 wherein said fragment of DNA is derived from a Rhizobium species.

7. A recombinant DNA plasmid as recited in Claim 5 comprising additionally a structural gene inserted in such orientation and location as to be expressible under control of said fragment of DNA.

8. A recombinant DNA plasmid as recited in Claim 7 wherein said structural gene is a foreign gene.

9. A method for expressing a structural gene under control of a Rhizobium species nif gene promoter fragment comprising the steps:

(a) isolating said nif gene promoter fragment,

(b) cloning said nif gene promoter fragment into a wide host range plasmid producing a recombinant DNA plasmid,

(c) isolating a DNA fragment carrying foreign structural genes and inserting said DNA fragment into said recombinant DNA plasmid at a position on the 3'-side of the reading strand of said nif promoter fragment giving a co-integrated recombinant plasmid, wherein said DNA fragment is oriented with respect to said promoter as to be expressible under control thereof,

(d) transforming said co-integrated recombinant plasmid into a bacterial species capable of a symbiotic relationship with plant cells, and

(e) infecting a legume plant with said bacterial species wherein expression of mRNA or protein coded by said foreign structural gene occurs.

10. A method as recited in Claim 9 wherein said nif gene promoter fragment is a Rhizobium species nifH promoter fragment.

11. A method as recited in Claim 9 wherein said nif gene promoter fragment is a Rhizobium species nifD promoter fragment.

12. A method as recited in Claim 9 wherein said bacterial species is a member of the genus Rhizobium.

13. A method for incorporating a DNA sequence into the chromosome of a gram-negative bacteria other than Escherichia coli comprising the steps;

(a) isolating a DNA fragment comprising the sequence to be incorporated,

(b) inserting said DNA fragment into a transposon carried on a suicide vector, said transposon having a selectable resistance gene contained within said transposon, to give a recombinant DNA plasmid,

(c) transforming said recombinant DNA plasmid into a first strain of bacteria capable of mobilizing said suicide vector into other gram-negative bacteria,

(d) transferring said recombinant DNA plasmid from said first strain of bacteria to a second strand of gram-negative bacteria by bacterial conjugation,

(e) growing said second strain under conditions requiring the presence of said selectable resistance gene, and

(f) selecting colonies of said second strain obtained from step (e) wherein said DNA fragment comprising the sequence to be incorporated is associated with chromosomal DNA.

14. A method as recited in Claim 13 wherein said appropriate DNA fragment is derived from a genome of a species of the genus Rhizobium.

15. A method as recited in Claim 13 wherein said transposon with said selectable resistance gene is transposon Tn5 with a kanamycin resistance gene.

16. A method as recited in Claim 13 wherein said gram-negative bacteria are a species of the genus Rhizobium.

FIG. 1a

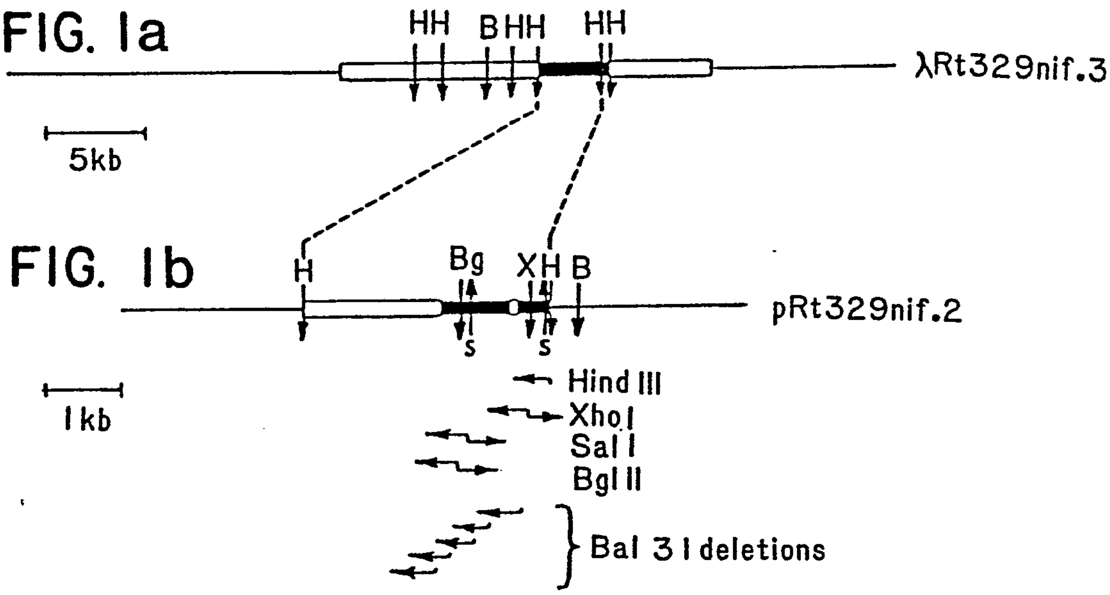


FIG. 1b

FIG. 3a

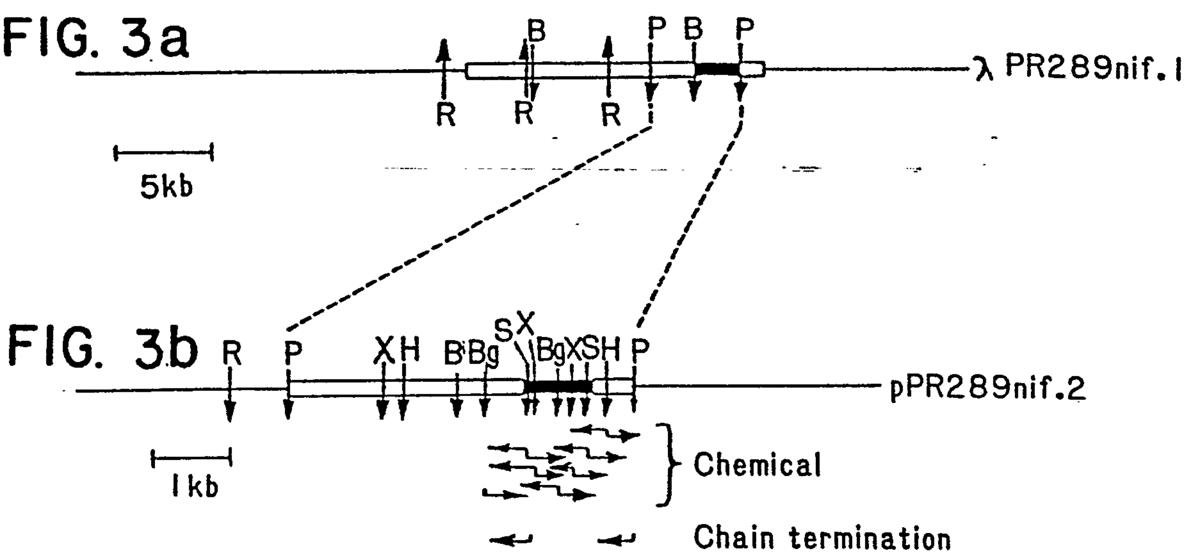


FIG. 3b

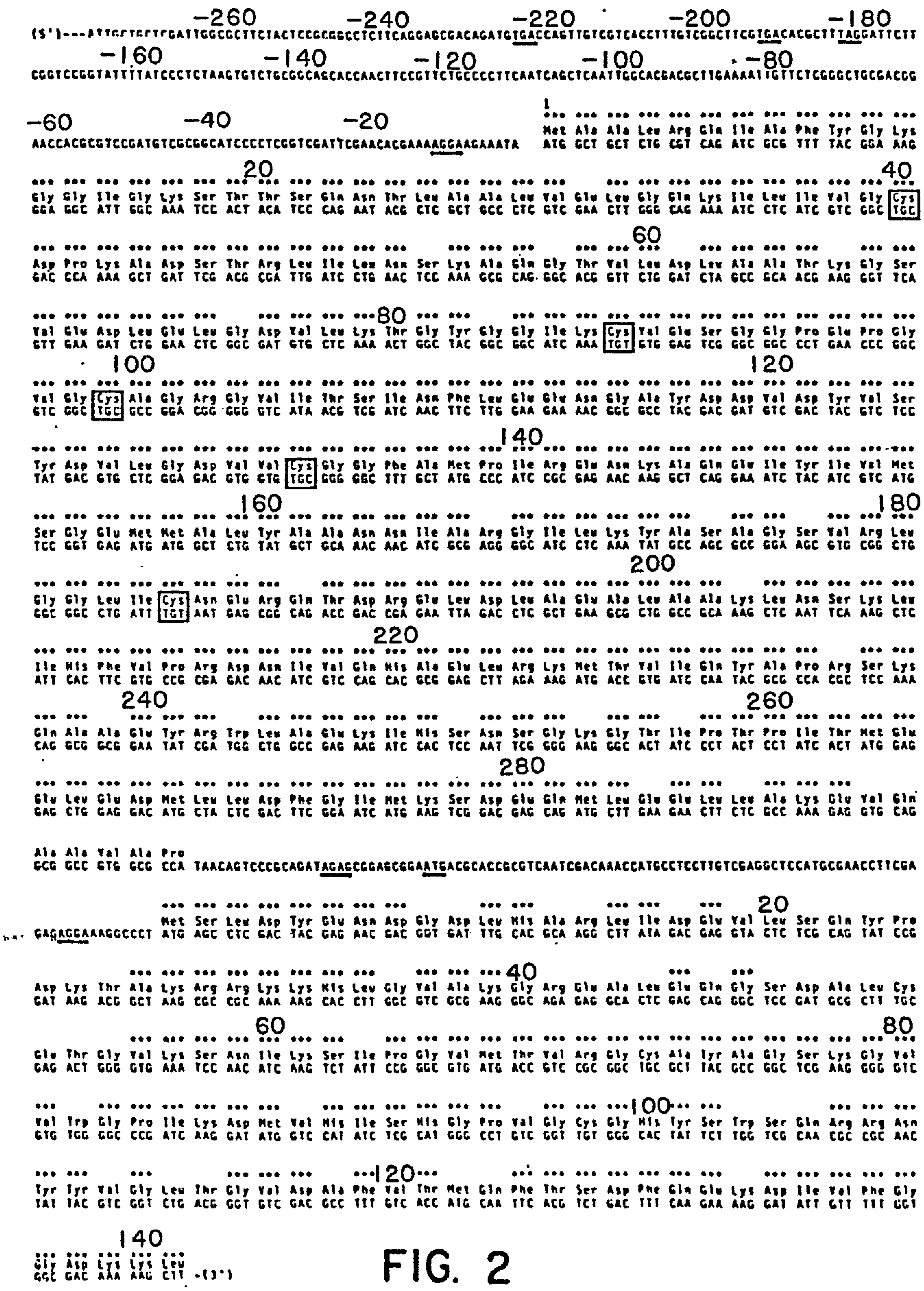

FIG. 2

# FIG. 4

(5') −400 −350
CTGCACGGCCCCTTGTAAGGCCGCTTCTTGCTGCCTTTAAGCTCATGCGGCACCGATCTGATCAGCTGGATCAATCGGGAGGTCAGCCCGCACAATTGATCTCGTCATCCTCGAC

−300 −250 −200
CACGAACCCCATCGCCCGGCCACTTGCCTTGAGGTTCTGACCTCGACCTGCATATTGCTCTCCGCCGGATTGCCCGCCACTGGCTTGCAAGAAGAGGAGCAAGTCCCGTTCCAG

−150 −100
TTGAGGAAATCGAACCAGATCATGCCAAACCGGCCGTTTTCCGGTTGATGGGTGTGGCCCGTTGTTCGTTTTCTGACAGCCCGCCAGATCCTGTCCGGTGCAAACCTCCCTCG

−50 −30 −10 20
GGTAGCTCAGCCGGCTCGTTGGCTTTTTAGAGCGTAATCAAGAAGCTTAATAAGCCGCGGACAGTGTTGGCATGGCCGATTGCCTGTTGAGTTGCAGCAACACTGAGTGAGGGCT

40 60 80 100 120
CGGTGCCACGCCCGACGCGTAAGACGAGCCGATGCCGCTCCTTCCCTTGAACCCGTGTGCCCCCGTTTCTGAGAGAGAAACAAGCTCGCCGTGTCGGAAGCACCGCA/.CTTTTGGCAA

140
ATCGGTTGATGGAGAACAAC

1 20
Met Ser Ser Leu Arg Gln Ile Ala Phe Tyr Gly Lys Gly Gly Ile Gly Lys Ser Thr Thr Ser Gln
ATG TCT TCA CTG AGA CAA ATC GCG TTC TAC GGA AAG GGC GGC ATC GGC AAG TCG ACC ACG TCC CAG

40
Asn Thr Leu Ala Ala Leu Ala Glu Met Gly Gln Lys Ile Leu Ile Val Gly Cys Asp Pro Lys Ala Asp Ser Thr Arg Leu Ile
AAT ACG TTG GCG GCA CTG GCC GAG ATG GGC CAG AAA ATC CTG ATC GTG GGA TGC GAT CCT AAG GCG GAC TCG ACG CGC CTC ATC

60
Leu His Ala Lys Ala Gln Asp Thr Ile Leu Ser Leu Ala Ala Ser Ala Gly Ser Val Glu Asp Leu Glu Leu Glu Asp Val Met
CTG CAC GCG AAG GCG CAG GAC ACG ATT TTG AGC CTT GCA GCG AGC GCT GGC AGC GTG GAA GAC CTC GAA CTC GAG GAC GTG ATG

80 100
Lys Val Gly Tyr Lys Asp Ile Arg Cys Val Glu Ser Gly Gly Pro Glu Pro Gly Val Gly Cys Ala Gly Arg Gly Val Ile Thr
AAG GTC GGC TAC AAG GAC ATC CGA TGC GTG GAG TCC GGT GGT CCC GAG CCG GGT GTC GGC TGC GCG GGC CGC GGC GTC ATC ACC

120
Ser Ile Asn Phe Leu Glu Glu Asn Gly Ala Tyr Glu Asn Ile Asp Tyr Val Ser Tyr Asp Val Leu Gly Asp Val Val Cys Gly
TCG ATC AAT TTC CTG GAG GAG AAC GGC GCC TAT GAG AAC ATT GAC TAT GTC TCA TAT GAC GTG CTC GGC GAC GTC GTT TGC GGT

140 160
Gly Phe Ala Met Pro Ile Arg Glu Asn Lys Ala Gln Glu Ile Tyr Ile Val Met Ser Gly Glu Met Met Ala Met Tyr Ala Ala
GGC TTT GCG ATG CCG ATC CGG GAA AAC AAG GCG CAG GAG ATC TAT ATC GTG ATG TCT GGA GAA ATG ATG GCA ATG TAT GCC GCA

180
Asn Asn Ile Ser Lys Gly Ile Leu Lys Tyr Ala Asn Ser Gly Gly Val Arg Leu Gly Gly Leu Ile Cys Asn Glu Arg Gln Thr
AAC AAT ATC TCC AAA GGT ATC CTG AAA TAC GCC AAC TCT GGC GGC GTG CGG CTG GGC GGC CTG ATC TGC AAC GAG CGG CAG ACC

200
Asp Lys Glu Leu Glu Leu Ala Glu Ala Leu Ala Lys Lys Leu Gly Thr Gln Leu Ile Tyr Phe Val Pro Arg Asp Asn Val Val
GAT AAG GAG CTC GAG CTG GCG GAG GCG CTG GCC AAG AAG TTA GGT ACT CAG CTG ATC TAC TTC GTG CCG CGC GAC AAT GTG GTG

220 240
Gln His Ala Glu Leu Arg Arg Met Thr Val Leu Glu Tyr Ala Pro Glu Ser Gln Gln Ala Asp His Tyr Arg Asn Leu Ala Thr
CAG CAT GCC GAG CTA CGG CGC ATG ACG GTG CTG GAG TAT GCG CCT GAG TCG CAG CAG GCC GAT CAC TAT CGC AAT CTT GCC ACC

260
Lys Val His Asn Asn Gly Gly Lys Gly Ile Ile Pro Thr Pro Ile Ser Met Asp Glu Leu Glu Asp Met Leu Met Glu His Gly
AAG GTT CAC AAC AAT GGC GGC AAA GGC ATC ATT CCG ACT CCG ATC TCC ATG GAT GAG CTC GAG GAC ATG CTG ATG GAG CAT GGC

280
Ile Met Lys Pro Val Asp Glu Ser Ile Val Gly Lys Thr Ala Ala Glu Leu Ala Ala Ser
ATT ATG AAG CCC GTC GAC GAA TCC ATC GTC GGC AAG ACC GCC GCC GAA CTC GCG GCC TCG TAAAGGTCGCGGGTCGCGGCCTTGTGAAGGC

GCGCCGACGGATGCCGGTCTCCCTCACCCCCCATCCGGGGAGACCGGCATTCTGACGATTATCTGACCAGCCAGAGTGGAGCTGGCAACCGTGACCGCTATGGCAACCCAAA

ACATCATGACAGGAGCGCACTTCCTTCCGCTTATGGCTTCTTGCGCCGTCGAGGCGAGCAGCAAGGTGCAAAGAGGAATTGCCGACCTACCGAGCGCTCACTGGCGTCCTCC

TGAAGAGGCCGACATTGCCGACCGACAGCAATTTCGATTGCCATGTCCTGGCCGTCAATCCTGGCGGCCGCTCGATGGATGGTGGCCCCGCTTCCCGAGCGCCCTGTCCGCCAC

CAGCTGGCCGACCCTGCTCGCAGCAATTTCCATCGGTTGAGGTCGATATCTCGGAGCAGCTCCTGGCCGTCTAAGTGCCGATGAGAATGACGAGATCGCCGATGGTGCCGCGATCT

TTTGCCTCAAGCAACGCTCGACGGACGGGCATATTCGGGCTGGCTAGCCGCCGATGATTGCCGCGCCGCGCCATAGAGCCAGATCACCTGTGGGAAGATCT.....(3')

FIG. 5 Parasponia Rhizobium ANU289 nif gene sequence.

```
2
AAT TCT CCG TCC AAA GCC CCA TTG CGC CTT CGC AAC AAC AAC CAG CCC CAT CGG ACG AAA
62
CGC GCT AAC TGT TTT ATT TAT TCT GCT TTT TGT GCC TCG CGC CGC GCT AAA CAT GCT CGT
122                                                                             1
                                                                        MET SER
TCC AGT CTT CTT CAA GAA GCT GCC CGC AAC AGT TAA TTC TTG AAG GAC ATC ACC ATG AGT
162                                                                            21
LEU ALA THR THR GLN SER ILE ALA GLU ILE ARG ALA ARG ASN LYS GLU LEU ILE GLU GLU
CTC GCC ACG ACC CAG AGC ATC GCA GAA ATC AGG GCT CGC AAT AAA GAG CTG ATC GAG GAG
242                                                                            41
VAL LEU LYS VAL TYR PRO GLU LYS THR ALA LYS ARG VAL ARG LYS HIS LEU ASN VAL HIS
GTG CTC AAA GTC TAT CCG GAG AAG ACC GCG AAA CGC GTG CGC AAG CAC CTC AAC GTT CAC
302                                                                            61
GLU ALA ALA GLY LYS SER ASP CYS GLY VAL LYS SER ASN ILE LYS SER ILE PRO GLY VAL
GAA GCC GCC GGC AAG TCG GAC TGC GGC GTC AAG TCC AAC ATC AAA TCA ATA CCT GGT GTG
362                                                                            81
MET THR ILE ARG GLY CYS ALA TYR ALA GLY SER LYS GLY VAL VAL TRP GLY PRO ILE LYS
ATG ACA ATC AGA GGC TGC GCC TAT GCA GGA TCC AAA GGC GTG GTC TGG GGA CCG ATC AAG
422                                                                           101
ASP MET VAL HIS ILE SER HIS GLY PRO VAL GLY CYS GLY GLN TYR SER TRP GLY SER ARG
GAC ATG GTC CAT ATC AGC CAT GGC CCG GTC GGC TGT GGT CAG TAT TCC TGG GGC TCG CGT
482                                                                           121
ARG ASN TYR TYR VAL GLY THR THR GLY VAL ASP SER PHE VAL THR LEU GLN PHE THR SER
CGC AAC TAT TAT GTT GGC ACG ACG GGC GTC GAT AGT TTC GTG ACC CTG CAG TTC ACC TCC
542                                                                           141
ASP PHE GLN GLU LYS ASP ILE VAL PHE GLY GLY ASP LYS LYS LEU ILE LYS VAL LEU ASP
GAC TTC CAG GAA AAG GAC ATC GTA TTT GGC GGC GAC AAG AAG CTG ATC AAA GTC CTT GAC
602                                                                           161
GLU ILE GLN GLU LEU PHE PRO LEU ASN ASN GLY ILE THR ILE GLN SER GLU CYS PRO ILE
GAA ATC CAG GAG CTG TTC CCG CTC AAC AAC GGC ATC ACC ATC CAA TCG GAA TGC CCG ATC
662                                                                           181
GLY LEU ILE GLY ASP ASP ILE GLU ALA VAL SER ARG SER LYS SER LYS GLU TYR GLY GLY
GGA CTG ATC GGC GAC GAC ATC GAG GCT GTG TCA AGA TCG AAA TCC AAA GAA TAC GGC GGC
722                                                                           201
LYS THR ILE VAL PRO VAL ARG CYS GLU GLY PHE ARG GLY VAL SER ARG SER LEU GLY HIS
AAG ACC ATC GTG CCT GTT CGC TGT GAG GGC TTT CGC GGC GTG TCA AGA TCG CTT GGC CAC
702                                                                           221
HIS ILE ALA ASN ASP ALA VAL ARG LEU ILE PHE ASP LYS LEU GLU PRO GLU ALA GLY PRO
CAC ATT GCC AAT GAC GCG GTG CGA TTG ATC TTC GAC AAG CTA GAG CCC GAG GCA GGA CCA
842                                                                           241
LYS PHE GLN PRO THR PRO TYR ASP VAL ALA ILE ILE GLY ASP TYR ASN ILE GLY GLY ASP
AAG TTC CAG CCG ACG CCC TAC GAC GTT GCG ATC ATC GGA GAC TAC AAT ATT GGC GGC GAT
902                                                                           261
ALA GLY SER SER HIS LEU LEU GLU GLU MET GLY LEU ARG VAL ILE ALA GLN TRP SER GLY
GCT GGG TCA TCG CAT TTG CTG GAA GAA ATG GGC TTG CGC GTG ATT GCG CAG TGG TCC GGC
962                                                                           281
ASP GLY SER LEU ALA GLU LEU GLU ALA THR ALA LYS ALA LYS LEU ASN ILE LEU HIS CYS
GAC GGT TCC CTC GCC GAA CTC GAA GCA ACC GCG AAG GCA AAG CTC AAT ATT CTG CAT TGC
1022                                                                          301
TYR ARG SER MET ASN TYR ILE SER ARG HIS MET GLU GLU LYS PHE GLY ILE PRO TRP CYS
TAC CGT TCC ATG AAC TAC ATC TCC CGC CAC ATG GAG GAG AAG TTT GGC ATC CCC TGG TGC
1082                                                                          321
GLU TYR ASN PHE PHE GLY PRO SER LYS ILE ALA GLU SER VAL ARG LYS ILE ALA GLY TYR
GAG TAC AAC TTC TTC GGA CCG TCG AAG ATC GCA GAA TCC GTG CGC AAG ATT GCG GGC TAT
1142                                                                          341
PHE ASP ASP LYS ILE LYS GLU ALA GLN ARG VAL ILE GLU LYS TYR SER THR GLY GLY ARG
TTC GAC GAC AAG ATC AAG GAA GCC CAG CGA GTA ATT GAA AAA TAC AGC ACT GGT GGA CGC
1202                                                                          361
ARG ASN ARG LYS ILE SER PRO ARG LEU GLU GLY LYS THR VAL MET LEU TYR VAL GLY GLY
CGT AAT CGC AAA ATA TCG CCC CGC CTG GAG GGC AAG ACT GTC ATG CTC TAC GTC GGC GGG
1262                                                                          381
LEU ARG PRO ARG HIS VAL ILE GLY ALA TYR GLU ASP LEU GLY MET GLU VAL VAL GLY THR
CTT CGT CCA CGT CAT GTG ATT GGC GCG TAC GAG GAT CTC GGC ATG GAA GTC GTG GGC ACC
1322                                                                          401
GLY TYR GLU PHE GLY HIS ASN ASP ASP TYR GLN ARG THR ALA GLN HIS TYR VAL LYS ASP
GGA TAC GAG TTC GGC CAC AAC GAC GAT TAT CAG CGC ACC GCC CAG CAC TAC GTT AAG GAC
1362                                                                          421
SER THR LEU ILE TYR ASP ASP VAL ASN GLY TYR GLU PHE GLU ARG PHE VAL GLU LYS VAL
AGC ACG CTC ATC TAC GAC GAC GTC AAT GGC TAT GAA TTC GAG CGC TTC GTC GAA AAG GTC
```

# FIG. 6

Comparison of the nucleotide sequences of the regulatory regions of the nifH, nifD and nifK operon of Rhizobium trifoli, ANU329, the nifH operon of Parasponia Rhizobium sp. ANU289 and of the nifD operon of Parasponia Rhizobium sp. ANU289

```
R.t.     ATT GCT GCT CGA TTG GCG CTT CTA CTC CGC GGC CTC TTC
P.R.-H   CCG CGC AGA TCC TGT CCG GTG CAA ACC TCC CTG GGG TAG
P.R.-D

R.t.     AGG AGC GAC AGA TGT GAC CAG TTG TCG TCA CCT TTG TCG GCT
P.R.-H   CTC AGC GGC TCG TTG GCT TTT TAG AGC GTA ATC AAG AAG CTT
P.R.-D

R.t.     TCG TGA CAC GCT TTA GGA TTC TTC GGT CCG GTA TTT TAT CCC
P.R.-H   AAT AAG CGC GGA CAG TGT TGG CAT GGC GAT TGC TGT TGA GTT
P.R.-D                               AAT TCT CCG TGC AAA GCG CGA

R.t.     TCT AAG TGT CTG CGG CAG CAC CAA CTT CCG TTC TGC CCC
P.R.-H   GCA GCA ACA CTG AGT GAG GGC TGG GTG CAC GCC GAC GCG
P.R.-D   TTG CGC CTT CGC AAC AAC AAC CAG CCC CAT CGG ACG AAA

R.t.     TTC AAT CAG CTC AAT TGG CAC GAC GCT TGA AAA TTG TTC
P.R.-H   TAA GAC GAG CGA TGC GCT CCT TCC CTT GAA CCC GTG TGC
P.R.-D   CGC GCT AAC TGT TTT ATT TAT TCT GCT TTT TGT GGC TCG

R.t.     TCG GGG TGC GAC GGA ACC ACG CGT CCG ATG TCG CGG CAT CCC
P.R.-H   CCC GTT TCT GAG AGA GAA ACA AGC TCG CGT GTC GGA AGC ACG
P.R.-D   CGC CGC GCT AAA CAT GCT CGT TGC AGT CTT GTT CAA GAA GCT

                                                 Met
R.t.     CTC GGT CGA TTC GAA CAC GAA AAG GAA GAA ATA ATG
P.R.-H   CAA CTT TTG GCA AAT CGG TTG ATG GAG AAC AAC ATG
P.R.-D   GCC CGC AAC AGT TAA TTC TTG AAG GAC ATC AGC ATG
```

FIG. 7

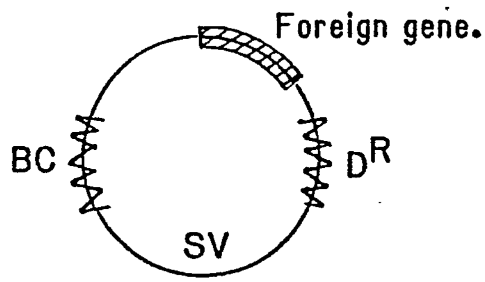


Co-integrated recombinant.

FIG. 8

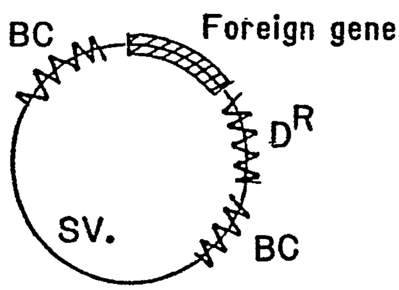


Co-integrated recombinant

FIG. 9

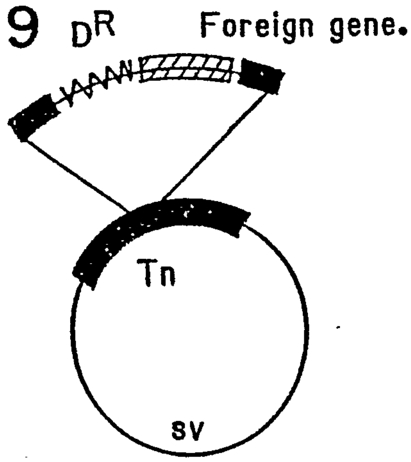


Co-integrated recombinant

FIG. 10 ANU289 nifH Regulatory Region.

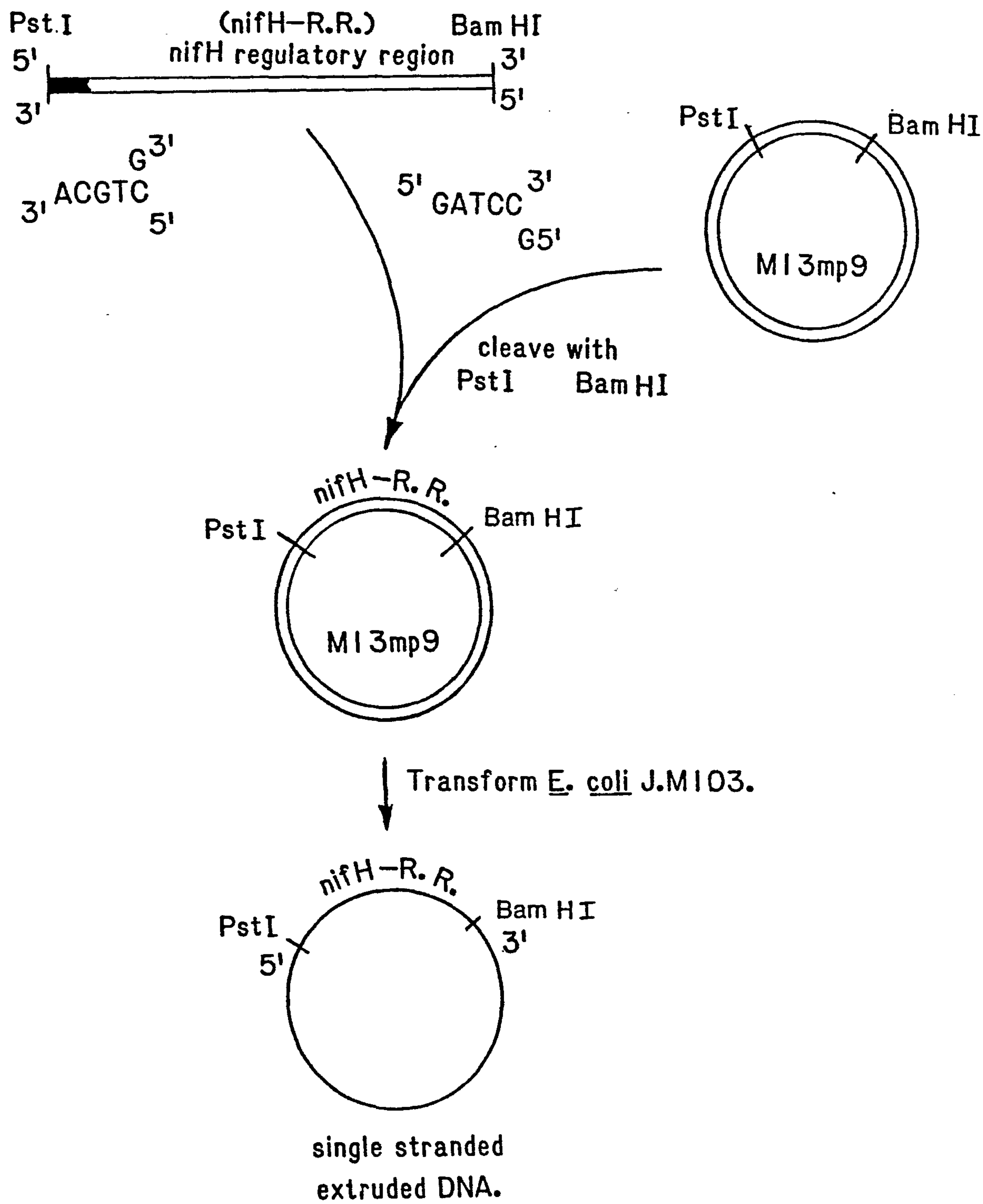

FIG. 11

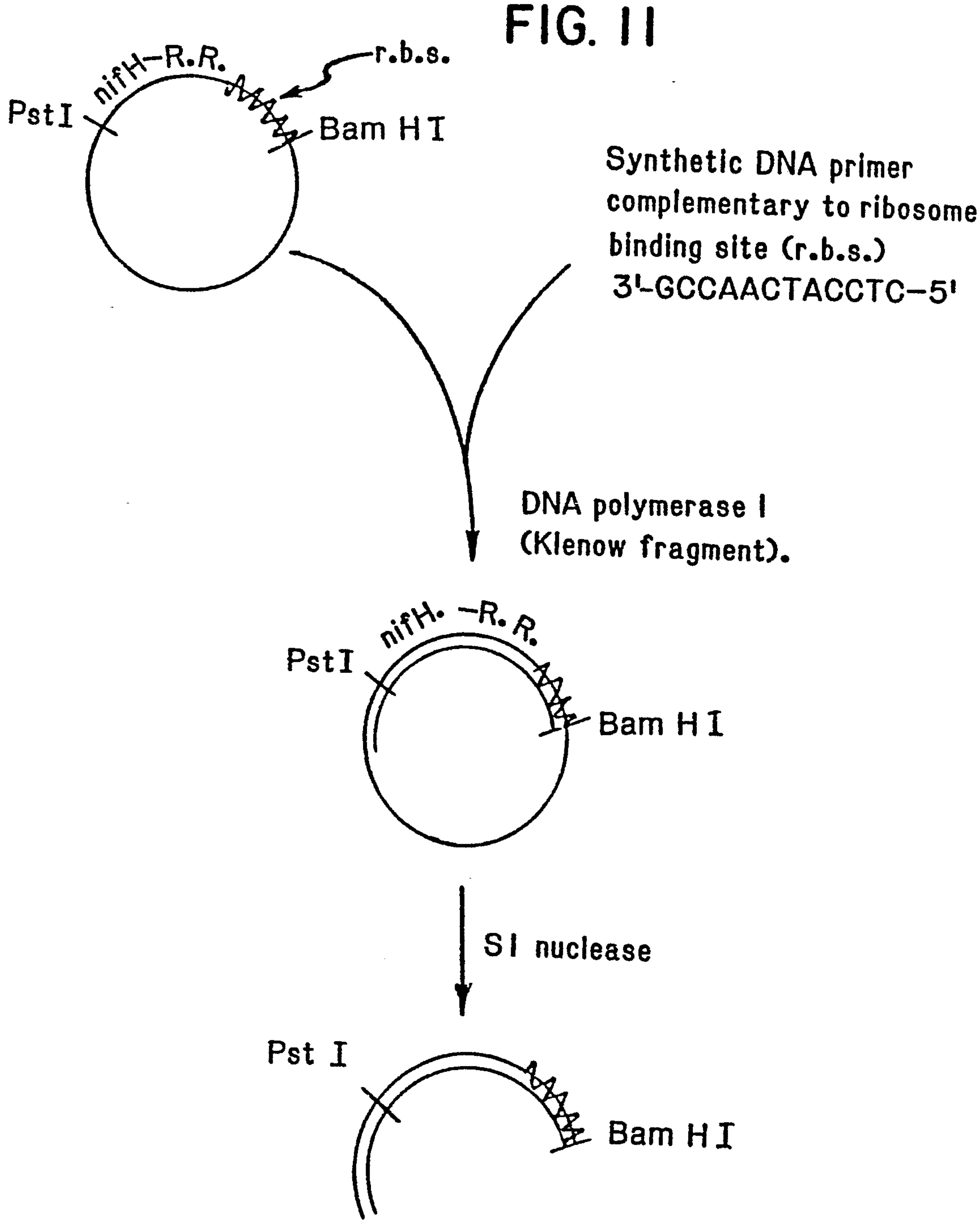
nifH–R.R.
r.b.s.
PstI
Bam HI
Synthetic DNA primer complementary to ribosome binding site (r.b.s.)
3'-GCCAACTACCTC-5'
DNA polymerase I (Klenow fragment).
nifH. –R. R.
PstI
Bam HI
SI nuclease
Pst I
Bam HI

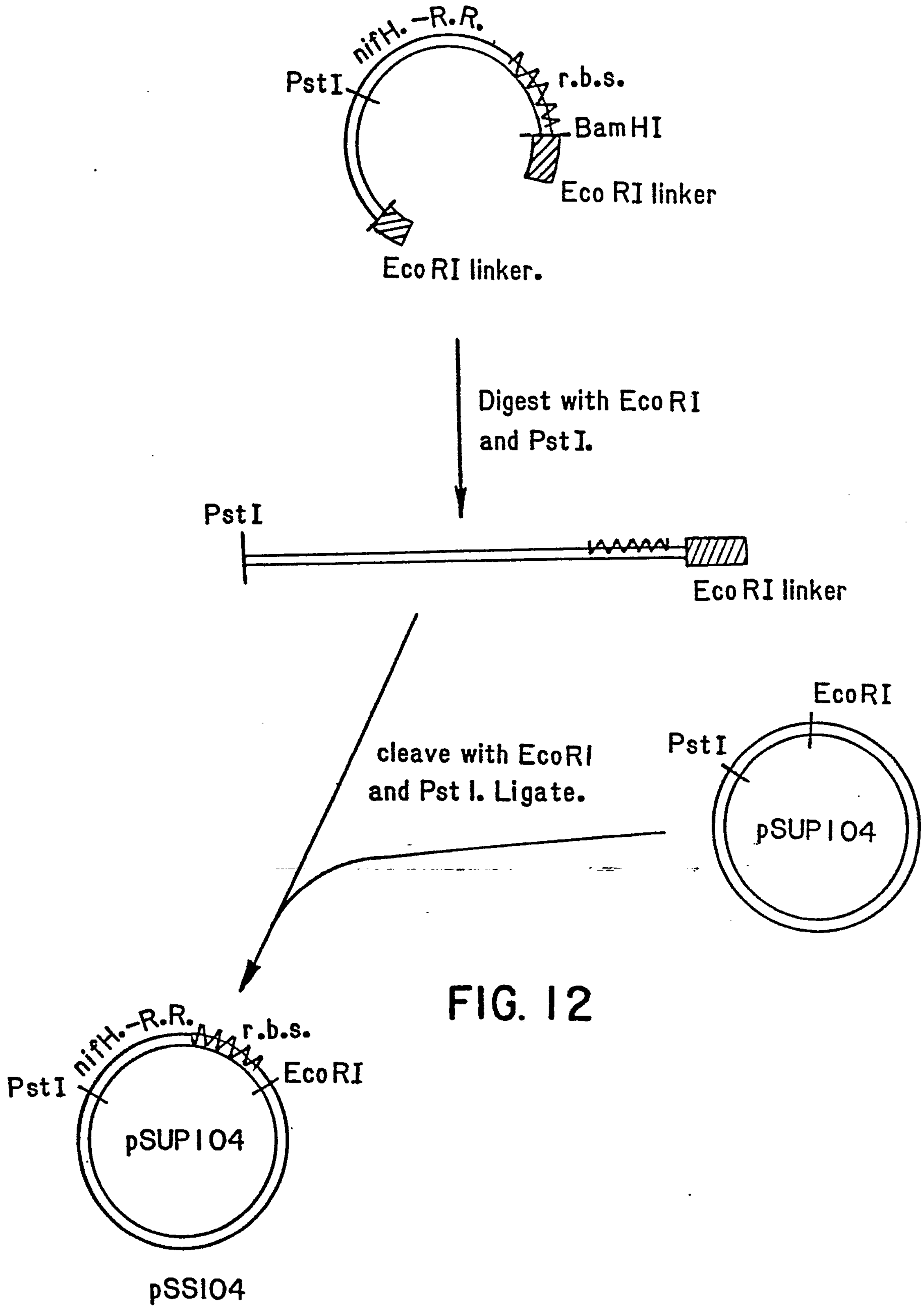
nifH.-R.R.
PstI
r.b.s.
BamHI
Eco RI linker
Eco RI linker.
Digest with EcoRI
and PstI.
PstI
Eco RI linker
EcoRI
PstI
cleave with EcoRI
and Pst I. Ligate.
pSUP104
nifH.-R.R.
r.b.s.
PstI
EcoRI
pSUP104
pSS104

FIG. 12

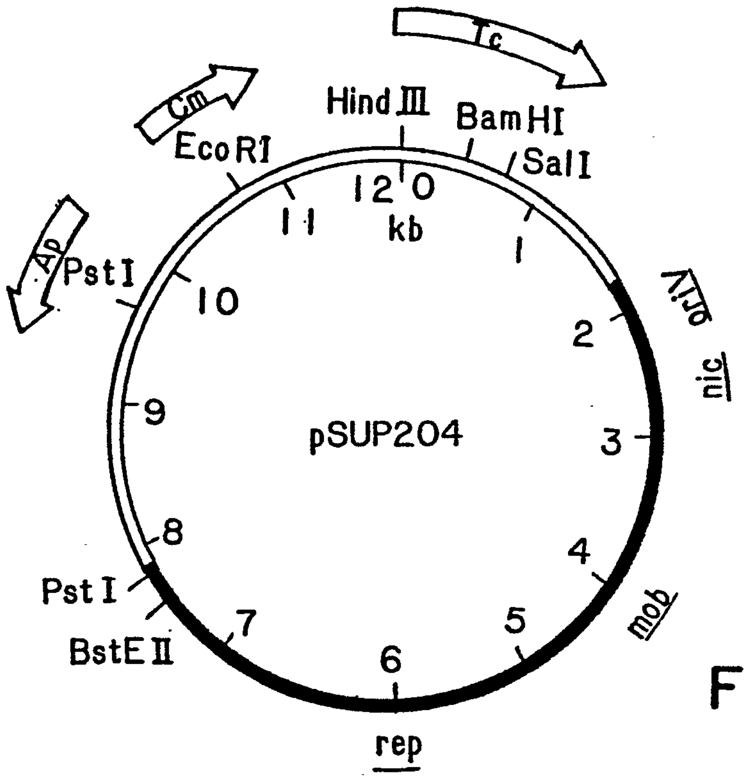


FIG. 13

PLASMID pSUP204 = pBR325 + (REP.MOB)

- approximate size is 12kb
- black region is derived from pKT210

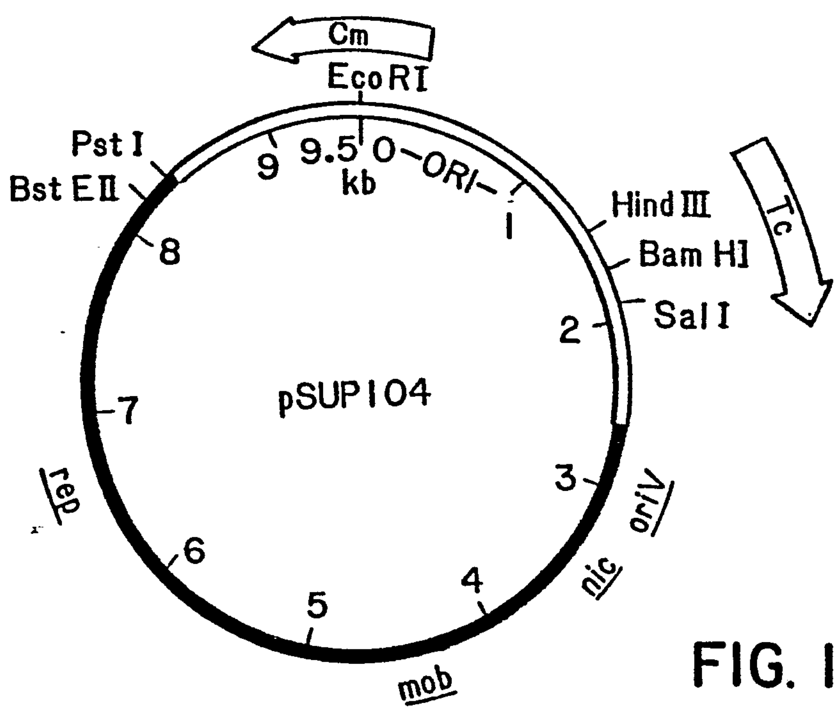


FIG. 14

PLASMID pSUP104 = pAC184 + (REP.MOB)

- size determined by electronmicroscopic investigation is 3.17 μm (±19%) (9.5 kb)
- the black region is derived from pKT210

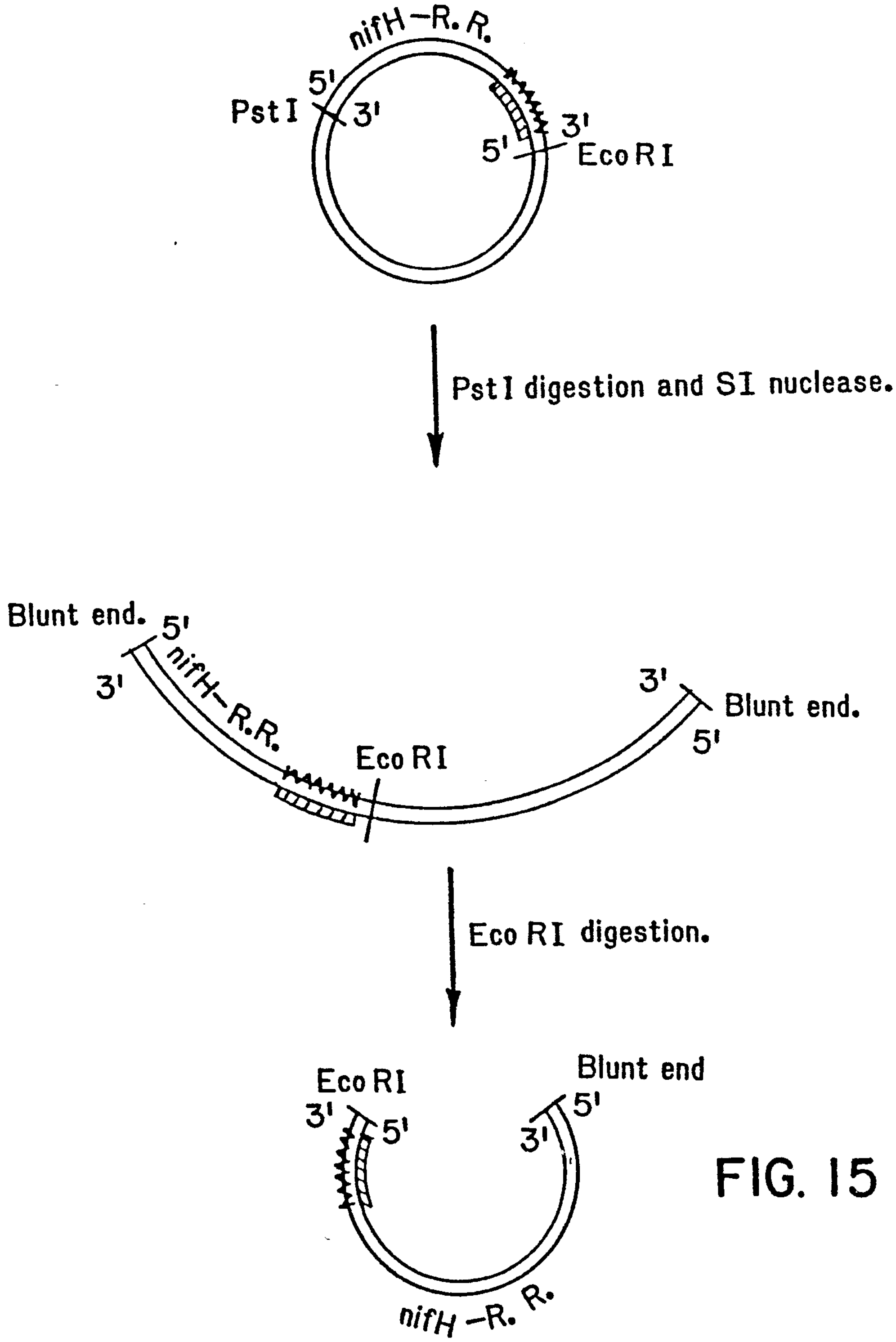
nifH-R.R.
5'
PstI
3'
3'
5'
EcoRI
PstI digestion and SI nuclease.
Blunt end.
5'
3'
nifH-R.R.
EcoRI
3'
Blunt end.
5'
EcoRI digestion.
EcoRI
3'
5'
Blunt end
5'
3'
nifH-R.R.

FIG. 15

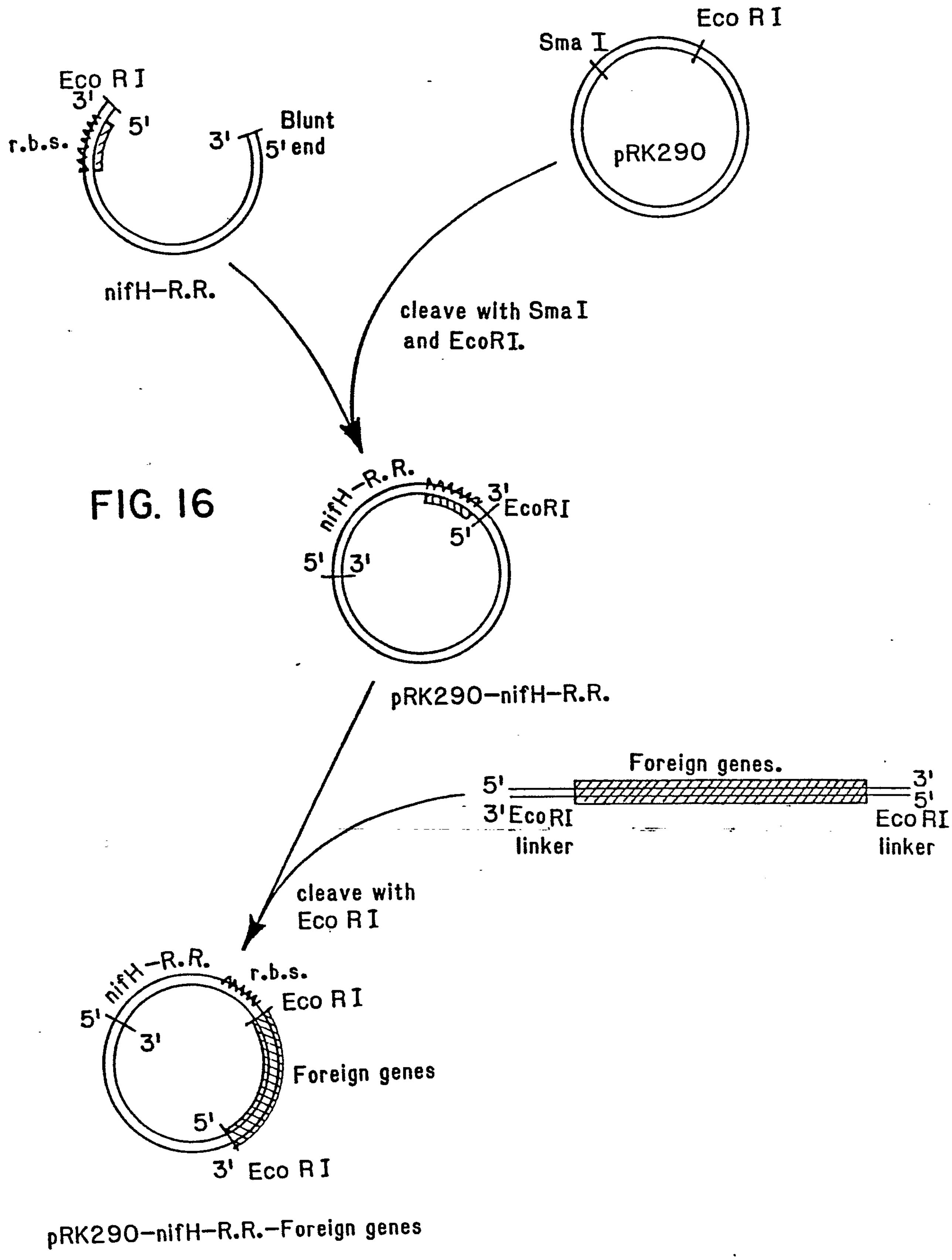
Eco RI
3'
r.b.s.
5'
3'
Blunt
5' end
nifH-R.R.
Sma I
Eco RI
pRK290
cleave with SmaI
and EcoRI.
nifH-R.R.
3'
EcoRI
5'
5'
3'
pRK290-nifH-R.R.
Foreign genes.
5'
3' EcoRI
linker
3'
5'
Eco RI
linker
cleave with
Eco RI
nifH-R.R.
r.b.s.
Eco RI
5'
3'
Foreign genes
5'
3' Eco RI
pRK290-nifH-R.R.-Foreign genes

FIG. 16

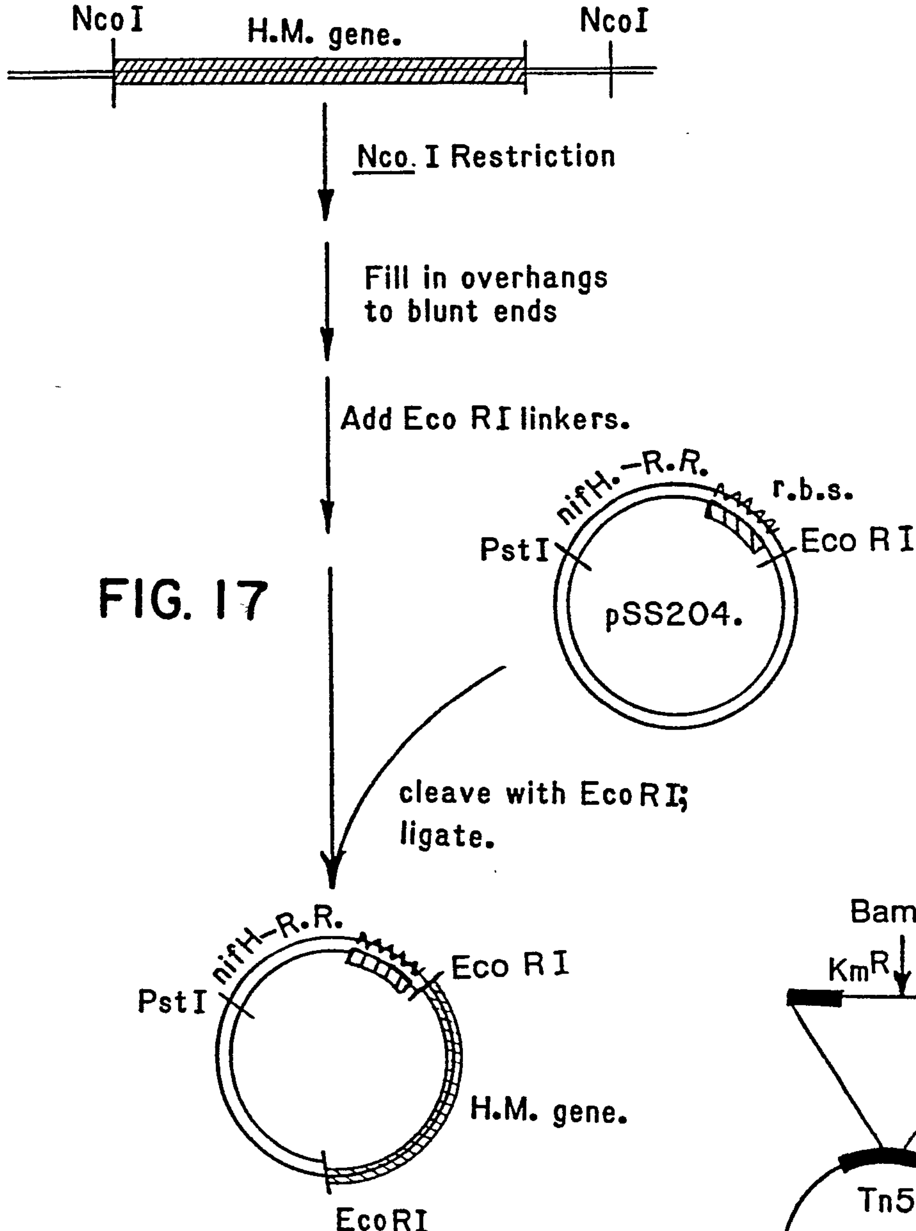
NcoI
H.M. gene.
NcoI
Nco. I Restriction
Fill in overhangs
to blunt ends
Add Eco RI linkers.
nifH.-R.R.
r.b.s.
PstI
Eco RI
pSS204.
cleave with EcoRI;
ligate.
nifH-R.R.
Eco RI
PstI
H.M. gene.
EcoRI

FIG. 17

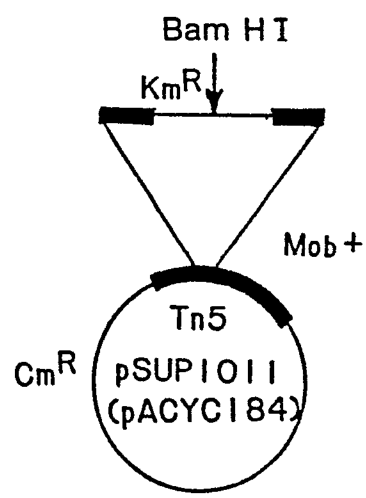
Bam HI
KmR
Mob+
Tn5
CmR
pSUP1011
(pACYC184)

FIG. 18

European Patent Office

# EUROPEAN SEARCH REPORT

0130047

Application number

EP 84304191.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | NATURE, vol. 288, no. 5786, November 6, 1980, London, New York<br>N.J. BREWIN et al. "Co-transfer of determinants for hydrogenase activity and nodulation ability in Rhizobium leguminosarum" pages 77-79<br>* Totality *<br>-- | 1-3,5, 6,9-12 | C 12 N 15/00//<br>C 12 R 1/41 |
| A,D | CURRENT PERSPECTIVES IN NITROGEN FIXATION, Proceedings of the Fourth International Symposium on Nitrogen Fixation held in Canberra, Australia December 1-5, 1980, Australian Academy of Science, Canberra, 1981<br>C. KENNEDY et al. "Recent advances in the Genetics and Regulation of Nitrogen Fixation" pages 146-156<br>* Totality *<br>-- | 1,5,9 | TECHNICAL FIELDS SEARCHED (Int. Cl. 3)<br>C 12 N |
| A,D | PROCEEDINGS OF THE NATIONAL ACADEMY OF SCIENCES OF THE UNITED STATES OF AMERICA, vol. 76, no. 6, June 1979, Baltimore, USA<br>G.E. RIEDEL et al. "Physical map of chromosomal nitrogen fixation (nif) genes of Klebsiella pneumoniae" pages 2866-2870<br>* Abstract *<br>---- | 1,5,9 | |

The present search report has been drawn up for all claims

| Place of search | Date of completion of the search | Examiner |
|---|---|---|
| VIENNA | 04-09-1984 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document